# EUROPEAN PATENT APPLICATION

(11) **EP 3 115 961 A1**
(43) Date of publication of application: **11.01.2017**
(21) Application number: 15757920.2
(22) Date of filing: 24.02.2015
(51) Int. Cl.: G06Q 50/22, G06K 7/10

(54) **GENE/CHROMOSOME TEST MANAGEMENT SYSTEM, TEST MANAGEMENT SERVER, CLIENT TERMINAL, METHOD FOR GENE/CHROMOSOME TEST MANAGEMENT, AND PROGRAM**

(30) Priority: 07.03.2014 JP 2014045218
(71) Applicant: FUJIFILM Corporation, Tokyo 106-8620 (JP)
(72) Inventor: KITAGAWA, Yusuke, Ashigarakami-gun Kanagawa 258-8538 (JP); SUGIHARA, Ryoichi, Ashigarakami-gun Kanagawa 258-8538 (JP)
(74) Representative: Klunker . Schmitt-Nilson . Hirsch
(86) International application number: PCT/JP2015/055215
(87) International publication number: WO 2015/133335

(57) **Abstract**

Provided are a genetic chromosome test management system, a test management server, a client terminal, a genetic chromosome test management method, and a program in which it is possible to reliably finish a test within a range in which a problem regarding the accuracy of the test is not caused, or it is possible to secure or ascertain the accuracy of the test. The genetic chromosome test management system (10) used for a genetic chromosome test for testing an abnormality of a chromosome contained in fetus-derived nucleated red blood cells includes: an identification information acquisition unit (14) which acquires identification information of a maternal body; a blood collection date and time specification unit (16) which specifies a blood collection date and time; a storage unit (20) which stores the identification information of the maternal body and information of the blood collection date and time; a test progress detection unit (24) which detects a progress condition of a test until at least a specific step one step before a step which is determined as not being affected by removal of nuclei; and a management unit (26) which manages a progress of the test until at least the specific step is completed, based on the detected progress condition.

## Description

### BACKGROUND OF THE INVENTION

### 1. Field of the Invention

The present invention relates to a genetic chromosome test management system, and specifically relates to a genetic chromosome test management system which is suitable for process management or work support of a genetic chromosome test for testing an abnormality of a chromosome from fetus-derived nucleated red blood cells, a test management server, a client terminal, a genetic chromosome test management method, and a program.

### 2. Description of the Related Art

A method for testing for an abnormality of a chromosome from fetus-derived nucleated red blood cells (NRBC) contained in maternal blood has been proposed as a non-invasive prenatal genetic test (NIPT) technique (JP2010-525832A and WO2012/023298A).

In JP2010-525832A, a method for performing genetic analysis by isolating fetus-derived nucleated red blood cells from blood collected from a pregnant woman is disclosed. In WO2012/023298A, a method for concentrating fetus-derived nucleated red blood cells contained in maternal blood to efficiently collect the fetus-derived nucleated red blood cells is proposed.

### SUMMARY OF THE INVENTION

It is known that nucleated red blood cells become red blood cells through removal of nuclei. Nucleated red blood cells of a fetus in a maternal body of a pregnant woman are always generated and flow into the maternal body. Therefore, there are nucleated red blood cells in a certain volume in blood circulating through the maternal body.

However, when implementing a specimen test outside the body after collecting blood from a maternal body, nucleated red blood cells are not newly generated in the blood after being collected, and nuclei are removed from the nucleated red blood cells in the blood over time. Therefore, the number of nucleated red blood cells in blood as a specimen gradually decreases. There is a problem in that a decrease in the number of nucleated red blood cells causes a problem regarding the accuracy of the success or failure of a test.

Regarding this problem, it is desirable that a test is finished within a certain period of time. However, it can also be assumed that the procedure after collecting blood up until a test, or the time required for each step of the test will be different in each hospital or each test room.

In the related art, in either of a case where the procedure after collecting blood up until a test, or the time required for each step of the test is different or the same in each hospital or each test room, effective means which can secure accuracy of testing has not been proposed.

The present invention has been made in consideration of such circumstances, and an object of the present invention is to provide a genetic chromosome test management system, a test management server, a client terminal, a genetic chromosome test management method, and a program in which it is possible to reliably finish a test within a range in which a problem regarding the accuracy of the success or failure of the test is not caused, or it is possible to secure or ascertain the accuracy of the success or failure of the test.

The following invention aspects are provided in order to achieve the purpose.

A genetic chromosome test management system according to a first aspect is a genetic chromosome test management system used for a genetic chromosome test for testing an abnormality of a chromosome contained in fetus-derived nucleated red blood cells, the genetic chromosome test management system comprising: an identification information acquisition unit which acquires identification information of a maternal body; a blood collection date and time specification unit which specifies a date and time at which blood is collected from the maternal body; a storage unit which stores the identification information of the maternal body acquired from the identification information acquisition unit and information of the blood collection date and time specified by the blood collection date and time specification unit in association with each other; a test progress detection unit which detects a progress condition of a test until at least a specific step is completed in a case where a step one step before a step which is determined as not being affected by removal of nuclei among a plurality of steps successively executed in the genetic chromosome test is regarded as the specific step; and a management unit which manages a progress of the test until at least the specific step is completed, based on the progress condition detected by the test progress detection unit.

According to the first aspect, the system manages the progress of the test until at least the specific step one step before the step which is determined as not being affected by removal of nuclei is completed based on the blood collection date and time which has been specified. Accordingly, it is possible to perform the test while maintaining time limits until the specific step in which there is a possibility there have been effects due to removal of nuclei is completed. According to the first aspect, it is possible to secure or ascertain the accuracy of the success or failure of the test.

In addition, according to the first aspect, it is possible to maintain the quality of the test even in a case where a working method or a system which is different for each hospital or each test room is employed, by determining a uniform management rule of managing a progress of a test until at least the specific step is completed based on the blood collection date and time.

As a second aspect, in the genetic chromosome test management system of the first aspect, the management unit can be configured to manage an elapsed time from the blood collection date and time.

For example, the management unit can be configured to display a warning and to output other alerts for attracting attention in a case where the elapsed time from the blood collection date and time is monitored and exceeds a predetermined time.

As a third aspect, in the genetic chromosome test management system according to the second aspect, the management unit can be configured to manage the progress of the test of a blood specimen collected from the maternal body.

For example, it is possible to manage the progress of tests in a test center in which a specimen test is performed after receiving work consignments from a hospital or the like, and other test rooms.

As a fourth aspect, in the genetic chromosome test management system according to the third aspect, the management unit can be configured to manage at least the progress of the testing up until a step of immobilizing fetus-derived nucleated red blood cells among a plurality of steps, and the elapsed time from the blood collection date and time.

As a fifth aspect, in the genetic chromosome test management system according to the third aspect, the management unit can be configured to manage at least the progress of the testing up until a step of extracting deoxyribonucleic acid (DNA) from the fetus-derived nucleated red blood cells, and the elapsed time from the blood collection date and time.

As a sixth aspect, the genetic chromosome test management system according to any one of the third aspect to the fifth aspect can be configured such that: a critical elapsed time determined as a critical threshold value which is allowable as the elapsed time after the blood collection is stored in the storage unit; the management unit determines whether or not the test is completed based on the progress condition of the test in a case where the elapsed time exceeds the critical elapsed time by comparing the elapsed time with the critical elapsed time; and the genetic chromosome test management system further comprises a notification unit which notifies excess information showing that the elapsed time has exceeded the critical elapsed time in a case where it is considered that the test has not been completed from the determination of the management unit.

As a seventh aspect, the genetic chromosome test management system according to the sixth aspect can be configured such that, in a case where the excess information is notified, the management unit can change the order of tests of a blood specimen in which the tests are not completed.

As an eighth aspect, the genetic chromosome test management system according to the sixth aspect or the seventh aspect can be configured such that the critical elapsed time is set for each layer which is classified according to at least one of the maternal body conditions of the age of the maternal body or the number of weeks elapsed after pregnancy; and the management unit manages patient information including at least one of the age of the maternal body or the number of weeks elapsed after pregnancy, acquires the critical elapsed time based on the patient information, and compares the critical elapsed time with the elapsed time.

As a ninth aspect, the genetic chromosome test management system according to any one of the third aspect to the eighth aspect can be configured such that the progress of the test of the blood specimen is managed by a barcode; and the barcode is created when blood is collected from the maternal body.

As means in place of the barcode, it is also possible to use radio frequency identification (RFID).

As a tenth aspect, the genetic chromosome test management system according to the ninth aspect can be configured such that at least one of information pieces of the blood collection date and time or identification information of the maternal body is included in the barcode.

As an eleventh aspect, the genetic chromosome test management system according to any one of the second aspect to the tenth aspect can be configured to further comprise: a display unit which displays at least one of the blood collection date and time or the elapsed time.

As a twelfth aspect, the genetic chromosome test management system according to any one of the second aspect to the tenth aspect can be configured to further comprise: a printing unit which creates a printed matter in which the information of the blood collection date and time is printed.

As a thirteenth aspect, the genetic chromosome test management system according to any one of the first aspect to the twelfth aspect can be configured such that the blood collection date and time specification unit specifies a date and time at which a request for a test is received as the blood collection date and time.

A test management server according to a fourteenth aspect is a test management server used for a genetic chromosome test for testing an abnormality of a chromosome contained in fetus-derived nucleated red blood cells, comprising: a reception unit which receives identification information of a maternal body and information of a blood collection date and time at which blood is collected from the maternal body, from a first client terminal; a storage unit which stores the identification information of the maternal body and the information of the blood collection date and time in association with each other; and a management unit which receives detection information showing a progress condition of a test from a second client terminal including a test progress detection unit detecting the progress condition of the test until at least the specific step is completed in a case where a step one step before a step which is determined as not being affected by removal of nuclei among a plurality of steps successively executed in the genetic chromosome test is regarded as the specific step, and manages a progress of the test until at least the specific step is completed, based on the detection information.

A client terminal according to a fifteenth aspect is a client terminal used for a genetic chromosome test for testing an abnormality of a chromosome contained in fetus-derived nucleated red blood cells, comprising: an identification information acquisition unit which acquires identification information of a maternal body; a blood collection date and time specification unit which specifies a date and time at which blood is collected from the maternal body; and a transmission unit which transmits the identification information of the maternal body and the information of the blood collection date and time to a test management server, which manages the identification information of the maternal body acquired from the identification information acquisition unit and the information of the blood collection date and time specified by the blood collection date and time specification unit, and manages a progress of a test until at least a specific step is completed in a case where a step one step before a step which is determined as not being affected by removal of nuclei among a plurality of steps successively executed in the genetic chromosome test is regarded as the specific step.

The client terminal according to the fifteenth aspect can be installed in, for example, a hospital, and can be used as a client terminal on the hospital side.

A client terminal according to a sixteenth aspect is a client terminal used for a genetic chromosome test for testing an abnormality of a chromosome contained in fetus-derived nucleated red blood cells, comprising: a test progress detection unit which detects a progress condition of a test until at least a specific step is completed in a case where a step one step before a step which is determined as not being affected by removal of nuclei among a plurality of steps successively executed in the genetic chromosome test is regarded as the specific step; and a transmission unit which transmits information of the progress condition detected by the test progress detection unit to a test management server managing a progress of the test until at least the specific step is completed, based on the progress condition detected by the test progress detection unit.

The client terminal according to the sixteenth aspect can be installed in, for example, a test room in which work consignments of specimen tests are received, and be used as a client terminal on the test room side.

Matters specified in the second aspect to the thirteenth aspect can be appropriately combined with each of the aspects from the fourteenth aspect to the sixteenth aspect.

A genetic chromosome test management method according to a seventeenth aspect is a genetic chromosome test management method for managing a genetic chromosome test for testing an abnormality of a chromosome contained in fetus-derived nucleated red blood cells, comprising: an identification information acquisition step of acquiring identification information of a maternal body; a blood collection date and time specification step of specifying a date and time at which blood is collected from the maternal body; a storage step of storing the identification information of the maternal body acquired through the identification information acquisition step and information of the blood collection date and time specified through the blood collection date and time specification step in association with each other; a test progress detection step of detecting a progress condition of a test until at least a specific step is completed in a case where a step one step before a step which is determined as not being affected by removal of nuclei among a plurality of steps successively executed in the genetic chromosome test is regarded as the specific step; and a management step of managing a progress of the test until at least the specific step is completed, based on the progress condition detected through the test progress detection step.

The same matters as those specified by the second aspect to the thirteenth aspect can be appropriately combined with the seventeenth aspect. In this case, a processing unit or a functional unit as means for performing processing or a function specified in the genetic chromosome test management system can be ascertained as an element which is a "process (step)" of processing or an operation corresponding thereto.

A program according to an eighteenth aspect is a program for causing a computer to realize a genetic chromosome test management function used for a genetic chromosome test for testing an abnormality of a chromosome contained in fetus-derived nucleated red blood cells, the program causing the computer to realize: an identification information acquisition function of acquiring identification information of a maternal body; a blood collection date and time specification function of specifying a date and time at which blood is collected from the maternal body; a storage function of storing the identification information of the maternal body acquired from the identification information acquisition function and information of the blood collection date and time specified using the blood collection date and time specification function in association with each other; a test progress detection function of detecting a progress condition of a test until at least a specific step is completed in a case where a step one step before a step which is determined as not being affected by removal of nuclei among a plurality of steps successively executed in the genetic chromosome test is regarded as the specific step; and a management function of managing a progress of the test until at least the specific step is completed, based on the progress condition detected using the test progress detection function.

The same matters as those specified by the second aspect to the thirteenth aspect can be appropriately combined with the program according to the eighteenth aspect. In this case, a processing unit or a functional unit as means for performing processing or a function specified in the genetic chromosome test management system can be ascertained as an element which is a "function" of processing or an operation corresponding thereto.

According to the present invention, it is possible to obtain at least one effect that it is possible to reliably end a genetic chromosome test within a range in which a problem regarding the accuracy of the success or failure of the test is not caused, or it is possible to secure or ascertain the accuracy of the success or failure of the test.

### BRIEF DESCRIPTION OF THE DRAWINGS

Fig. 1 is a flowchart in which a procedure of a genetic chromosome test is shown.
Fig. 2 is a flowchart in which a flow of a work of a cell isolation step is shown.
Fig. 3 is a flowchart in which a flow of a work of a genetic analysis step is shown.
Fig. 4 is a conceptual view illustrating a method for dividing steps in a test process through generalization.
Fig. 5 is a block diagram in which an outline of a genetic chromosome test management system according to the present embodiment is shown.
Fig. 6 is a configuration diagram of a genetic chromosome test management system 60 according to a first example.
Fig. 7 is an explanatory view in which a state of executing a genetic chromosome test in the first example is schematically shown.
Fig. 8 is an explanatory view in which a state when providing a test result of the genetic chromosome test in the first example is schematically shown.
Fig. 9 is a view showing an example of a test result input screen.
Fig. 10 is a view showing an example of management information which is managed in a processing condition management server.
Fig. 11 is a view showing an example of a test result report confirmation screen.
Fig. 12 is an explanatory view in which a state when a new test is requested in the first example is schematically shown.
Fig. 13 is a conceptual view in which an example of a method for managing a progress of a test using the processing condition management server is shown.
Fig. 14 is a block diagram showing a configuration example of the processing condition management server.
Fig. 15 is a block diagram showing a configuration example of a hospital terminal.
Fig. 16 is a block diagram showing a configuration example of a test terminal.
Fig. 17 is a configuration view of a genetic chromosome test management system according to a second example.
Fig. 18 is a configuration view of a genetic chromosome test management system according to a third example.

### DESCRIPTION OF THE PREFERRED EMBODIMENTS

Hereinafter, an embodiment of the present invention will be described in detail in accordance with accompanying drawings.

### <Outline of Test Procedure of Genetic Chromosome Test>

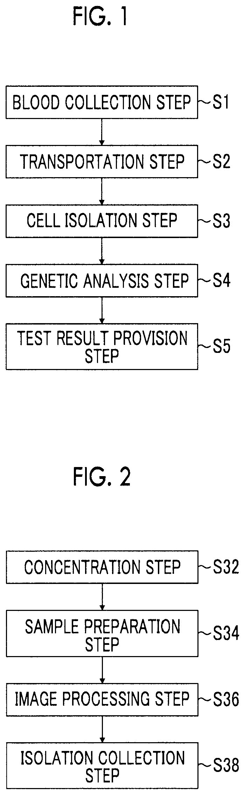

First, an overall flow of a procedure of a genetic chromosome test for testing an abnormality of a chromosome contained in fetus-derived nucleated red blood cells will be outlined. Fig. 1 is a flowchart in which a rough flow of a genetic chromosome test is shown. The genetic chromosome test has a blood collection step S1, a transportation step S2, a cell isolation step S3, a genetic analysis step S4, and a test result provision step S5.

The blood collection step S1 is a step of collecting peripheral blood from a maternal body of a pregnant woman, and can be ascertained as a specimen collection step of collecting a blood specimen. The blood collection step S1 performs, for example, direct blood collection from a maternal body performed by a nurse or the like in a Gynecology hospital.

The transportation step S2 is a step of transporting the collected specimen in the hospital to a test room. The term test room is used as a collective name of a place, an institution, an agency, a company, or the like in which a work of testing a specimen is executed. For example, a test institution, a test center, a test company, a medical laboratory, or the like which is specialized for a work of testing a specimen after receiving work consignments from a medical institution such as a hospital is considered as the test room. In addition, the test room may be attached to a hospital.

The cell isolation step S3 is a step of isolating fetus-derived nucleated red blood cells from maternal blood of a blood specimen, and collecting the fetus-derived nucleated red blood cells.

The genetic analysis step S4 is an analysis step of analyzing a numerical abnormality of chromosomes of a fetus, from the isolated and collected nucleated red blood cells. In the genetic analysis step S4, analysis of detecting a numerical abnormality of chromosomes of a fetus is performed. As a numerical abnormality of chromosomes, the presence or absence of at least one of monosomy, trisomy, or tetrasomy is checked.

The test result provision step S5 is a step of reporting test results obtained through genetic analysis to a hospital side. The test results performed in the test room are electronically provided to the hospital side online as a test result report or are provided in a form of a printed matter thereto.

Fig. 2 is a flowchart in which a flow of the work of the cell isolation step is shown. The cell isolation step S3 (refer to Fig. 1) includes, for example, a concentration step S32, a sample preparation step S34, an image processing step S36, and an isolation collection step S38 as shown in Fig. 2.

The concentration step S32 is a step of concentrating nucleated red blood cells in peripheral blood of a maternal body of a pregnant woman. Fetus-derived nucleated red blood cells and maternal body-derived nucleated red blood cells are contained in peripheral blood of a maternal body. In the concentration step, the fetus-derived and the maternal body-derived nucleated red blood cells are concentrated. There is a density gradient centrifugal separation method, a removal method using a magnetic separation method of white blood cells, or the like as the concentration method.

The sample preparation step S34 is a step of preparing a smearing sample of the concentrated sample. In the sample preparation step S34, a smearing sample is prepared as a microscopic sample by spreading a concentrated liquid of a sample on slide glass and performing drying, fixing, dyeing, or the like. That is, the sample preparation step S34 includes a sample collection step, a sample smearing step, a first drying step, a dyeing step, and a second drying step when the sample preparation step is further divided.

The sample collection step is a step of collecting a sample which has been concentrated through a concentration step. The sample smearing step is a step of thinly and uniformly spreading the collected sample on slide glass. The first drying step is a step of drying the sample on the slide glass before the dyeing. The dyeing step is a step of performing dyeing of the sample which has been dried through the first drying step. As the dyeing method, it is possible to use Giemsa dyeing. The second drying step is a step of drying a sample after the dyeing.

A smearing sample is obtained through the sample collection step, the sample smearing step, the first drying step, the dyeing step, and the second drying step after the concentration step. In some cases, the term "smearing sample" is called a "slide sample" or is simply called a "slide".

The image processing step S36 is a step of acquiring a scan image of the smearing sample using a slide scanner, and detecting nucleated red blood cells by analyzing the obtained scan image. A method for detecting the shape of the nucleated red blood cells from the image or the like is used for detecting the nucleated red blood cells.

The isolation collection step S38 is a step of detecting the position of the nucleated red blood cells on the slide glass of the smearing sample based on the image analysis result of the scan image, and isolating and collecting the target nucleated red blood cells.

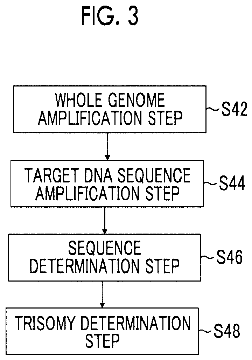

Fig. 3 is a flowchart in which the work of the genetic analysis step (S4 in Fig. 1) is shown. The genetic analysis step (S4 in Fig. 1) includes, for example, a whole genome amplification step S42, a target DNA sequence amplification step S44, a sequence determination step S46, and trisomy determination step S48 as shown in Fig. 3.

The whole genome amplification step S42 is a step of performing processing of amplifying whole genome from a single cell. About one or a few cells are insufficient for analysis, and therefore, DNA information is copied by about 10,000 times to several millions of times.

The target DNA sequence amplification step S44 performs processing of amplifying specific chromosome information from DNA desired to be amplified, using a polymerase chain reaction (PCR).

The sequence determination step S46 is a step of specifying attribution of chromosome using a next-generation sequencer to identify a base sequence of a maternal body and a base sequence of a fetus. That is, in the sequence determination step (S46), determination of quantification or attribution of DNA which has been amplified in the target DNA sequence amplification step S44 is performed.

The trisomy determination step S48 is a step of diagnosing a trisomy (a karyotype in which there are three chromosomes) which is a numerical abnormality of a specific autosome. Three chromosomes 13, 18, and 21 are currently considered as targets of a prenatal genetic test. In this example, diagnosis of "trisomy 21" which is a numerical abnormality of a 21st chromosome is performed.

The items of the steps described from Figs. 1 to 3 or the classification method of the steps show an example, and there is an arbitrariness in a dividing method in a series of test processes, that is, a step definition method for defining each of the steps as what kinds of contents. In addition, it is possible to employ various other methods for the test processes themselves, and similarly, there are also methods for defining the steps according to the test processes.

### <Regarding Necessity of Progress Management of Test Step>

The genetic chromosome test has a plurality of steps, and the work or the processing of each step is successively executed in order. As is already described, the number of nucleated red blood cells is gradually decreased over time after collecting blood. Originally, the number of nucleated red blood cells contained in maternal blood is small and the ratio of nucleated red blood cells existing in maternal blood is about 1/10⁵ to 1/10⁷ with respect to total nucleated cells, which is extremely small. In addition, the life cycle of nucleated red blood cells is short, and therefore, the number of nucleated red blood cells is further decreased due to removal of nuclei after collecting blood.

The small number of nucleated red blood cells causes a problem regarding the accuracy of the success or failure of a test. Therefore, it is necessary to manage the step of test so as to finish the test within a certain period of time after collecting blood from the viewpoint of securing accuracy of testing.

In the embodiment of the present invention, a genetic chromosome test management system is provided in which it is possible to reliably finish the test within a range in which there is no problem caused in accuracy of the success or failure of a test, or time management of a test capable of securing or ascertaining the accuracy of the success or failure of a test is performed.

The genetic chromosome test management system according to the present embodiment provides a mechanism which determines a step which is not affected by removal of nuclei, among a series of test processes of the genetic chromosome test, a progress condition of the test until at least a specific step prior to the step, which is determined as not being affected by removal of nuclei, is completed is ascertained, and the completion of the steps up until the specific step within a prescribed allowable time from the blood collection date and time is promoted, with respect to the problem in that the accuracy of testing is decreased in accordance with the decrease in the number of nucleated red blood cells due to removal of nuclei over time after collecting blood.

### <Description of Terms>

The method for dividing steps in a test process or the number of steps managed can be arbitrarily set. There is an arbitrariness in a method for determining a step which is not affected by removal of nuclei, and the step which is not affected by removal of nuclei is determined among a series of steps. In general, if nucleated red blood cells are immobilized, there is no possibility that nuclei are removed. Therefore, it is possible to determine the step of immobilizing nucleated red blood cells as the step which is not affected by removal of nuclei, among the test steps. The "immobilization" means that cells or organelles are made not to be moved in a form of a state in which the structures thereof are kept. There are both cases for the immobilization: a case of making structures of cells or organelles not to be moved while keeping the cell; and a case of making structures of cells when being alive after being killed.

For example, it is considered that nucleated red blood cells are immobilized in a stage in which a sample is smeared on slide glass and is dried through a slide preparing step after the sample collection step in the sample preparation step S34 described in Fig. 2. In this case, the drying step corresponds to an "immobilizing step" (immobilization step), a step after the drying step can be determined as the "step which is not affected by removal of nuclei". The step which is determined as the "step which is not affected by removal of nuclei" is a "step which is determined as not being affected by removal of nuclei".

In addition, it is considered that deterioration of cells is stopped by performing dyeing in the dyeing step of the sample preparation step S34. Therefore, it is possible to determine the dyeing step as the immobilization step and to determine a step after the dyeing step as the "step which is not affected by removal of nuclei".

All of the steps which are the first drying step, the dyeing step, and the second drying step of the sample preparation step S34 described in Fig. 2 can be a candidate which can be determined as the immobilization step. In addition, it is clear that at least there is no effect of removal of nuclei in the genetic analysis step S4 (refer to Fig. 1) after extracting DNA from nucleated red blood cells, and therefore, as another determination method, it is also possible to determine the isolation collection step S38 described in Fig. 2, a step of extracting DNA from nucleated red blood cells in the isolation collection step S38, or the genetic analysis step S4 (refer to Fig. 1) or the like as the "step which is not affected by removal of nuclei". For example, the step of extracting DNA from nucleated red blood cells can be set as a "specific step".

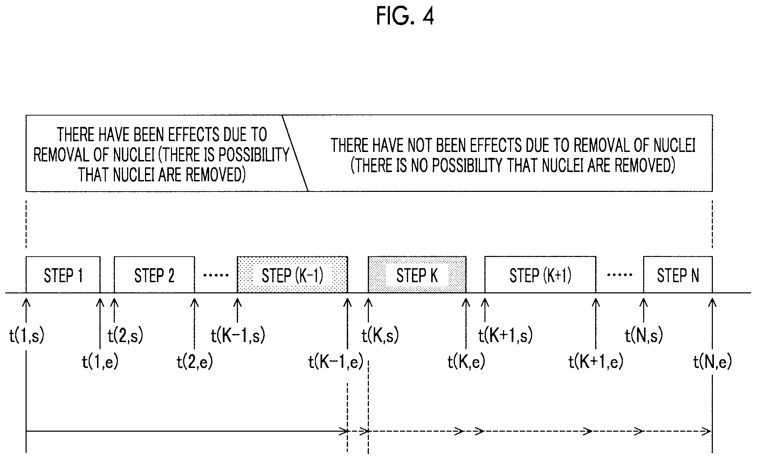

Fig. 4 is a conceptual view illustrating a method for dividing steps in a test process through generalization. Here, a case, in which a step number i indicating an execution order of a plurality of steps which have been successively executed is represented by integers from 1 to N, and a progress of the test process having steps in N stages from a step 1 to a step N is managed, will be described.

The lateral axis in Fig. 4 is a time axis. The steps from the step 1 to the step N are sequentially executed. In order to ascertain the progress of the test on the system, at least a time of a start time or an end time of a step is detected with respect to each step to be managed. The "time" of the start time or the completion time of a step means the date and time. The start date and time of a step refers to a "start time" and the completion date and time of a step refers to a "completion time".

The start time of the step i detected when starting a work or processing of the step i is denoted as t(i, s), and the completion time of the step i detected when completing the work or processing of the step i is denoted as t(i, e). For example, t(1, s) can be set to correspond to the blood collection date and time.

In Fig. 4, the start time and the end time of each step are written. However, it is possible to manage the steps by detecting at least one time. In addition, in Fig. 4, a state in which a time difference is provided between a completion time t(i, e) of a step i and a start time t(i+1, s) of the next step (i+1) is shown in the drawing. However, the completion time t(i, e) of the step i and the start time t(i+1, s) of the next step (i+1) may be coincident with each other. For example, in a case of detecting only the start time with respect to a certain step, it is ascertained that the completion time of the step is coincident with the start time of the next step in terms of management using the system.

A plurality of steps from the step 1 to the step N can be divided into two groups in view of whether a step receives or does not receive an effect of removal of nuclei. In Fig. 4, steps after a step K among the plurality of steps from the step 1 to the step N are determined as steps which are not affected by removal of nuclei by setting that steps after the K-th step K, which is a boundary, are not affected by removal of nuclei. The "K" is an integer of 2 to N. A step, which is one step before this step K which is determined as not being affected by removal of nuclei, that is, a step (K-1) which is a step immediately before the step K is called a "specific step". The specific step is a step which is relatively specified based on the relationship to the step K which is determined as not being affected by removal of nuclei. Moreover, the specific step corresponds to the last step in which there is a possibility that there have been effects due to removal of nuclei. The step K is a leading step of the steps which are determined as not being affected by removal of nuclei.

Regarding the method for determining the "step which is not affected by removal of nuclei", in a strict sense, it is unnecessary for the step K to actually be a step at a boundary at which the state of the step K is shifted from a state in which there is a possibility that nuclei are removed to a state in which there is no possibility that nuclei are removed. For example, in a case where there is no possibility where nuclei are removed in the middle of processing of the step (K-1) as shown in Fig. 4, it is possible to determine that "there have not been effects due to removal of nuclei" in the step K. However, instead of such a determination method, it is also possible to determine a step after the step (K+1) as the step which is not affected by removal of nuclei, while the step K is included in the step of receiving an effect of removal of nuclei. In a case where the step (K+1) is determined as a leading step of the "steps which are determined as not being affected by removal of nuclei", the step K immediately before the step (K+1) corresponds to the "specific step".

The meaning of being no possibility that nuclei are removed in the middle of the processing of the step (K-1) is that there are also a plurality of work processes in the step which has been determined as the step (K-1) and the removal of nuclei is suppressed in processing performed as a certain work in the work processes. As the management method of the system, it is not necessary for the system to be constructed so as to be able to detect the execution of such processing. Therefore, the step in which such processing is included as the work becomes the final step receiving an effect of removal of nuclei.

In the genetic chromosome test management system according to the embodiment of the present invention, the progress condition is ascertained until at least a specific step among the plurality of steps is completed. In a case where the step K is the "step which is determined as not being affected by removal of nuclei" as shown in Fig. 4, the progress condition is ascertained until at least the step (K-1) is completed.

In a case where the completion time t(K-1, e) of the step (K-1) is detected, the progress condition is ascertained up until at least t(K-1, e). In a case where the start time t(K, s) of the step K is detected without detecting the completion time t(K-1, e) of the step (K-1), the progress condition is ascertained up until at least t(K, s).

The ascertaining of the progress condition until the specific step is completed is, in other words, substantially equivalent to ascertaining of the progress condition until the "step which is determined as not being affected by removal of nuclei" is started. The ascertaining of the progress condition until the step (K-1) is completed substantially has the same meaning as the ascertaining of the progress condition immediately before the start of the step K.

In addition, in the example of Fig. 4, in a case where the steps after the step (K+1) are determined as steps which are not affected by removal of nuclei and the step K immediately before the step (K+1) is set as a "specific step", the management is strictly performed until the step K is completed. Therefore, in reality, in a case where processing of suppressing removal of nuclei is performed in the step (K-1), the work time of the step K becomes a margin in time management. That is, the significance of ascertaining the progress condition until the specific step in this case is completed is that it is possible to manage the progress including margin time.

If processing of suppressing removal of nuclei is included between the first step 1 in which blood is collected and a step of ascertaining and managing the progress condition, it is possible to secure accuracy of testing, which is the gist of the present invention. The significance of determining a specific step is that it is possible to perform flexible management regardless of a case where the processing of suppressing removal of nuclei is different in hospitals or test rooms, or the difference in the steps up until the processing.

When describing the difference in the steps, in general, the step N is a step of reporting test results. That is, in a case where, for example, N is 2, there are also hospitals or test rooms in which the progress condition is ascertained while gathering all of a series of processing before the step of reporting test results. In this case, an effect of removal of nuclei is not naturally received at a point in time of the step 2 of reporting test results, and therefore, the step 1 immediately before the step 2 is set to a specific step. However, in such a case, there is a problem in that the number of tests which is receivable when determining whether test requests are acceptable to be described below is decreased as much as a portion in which the margin is large. The trade-off between a setting for how much margin is provided or a setting for how degree the tests are received can be set so as to flexibly respond to different conditions according to work capacities of hospitals or test rooms. In addition, there is a possibility that the items of the steps described in Figs. 1 to 3 may also be changed in the future with advancement of technology. Therefore, it is important to have a system which can flexibly respond the different conditions in the future while securing the accuracy of the success or failure of a test.

It is possible to manage the progress even after the completion of the step (K-1) which is a specific step. The progress of all of the steps from the step 1 to the step N may be managed, or the progress of arbitrary steps from the step K to the step N may be managed.

Any of the detection of a progress condition of a test until a leading step of the steps which are determined as not being affected by removal of nuclei is completed, or the detection of a progress condition of a test until a step after the leading step of the steps which are determined as not being affected by removal of nuclei is included in a concept of "detecting progress condition of a test until at least the specific step is completed".

### <Outline of System>

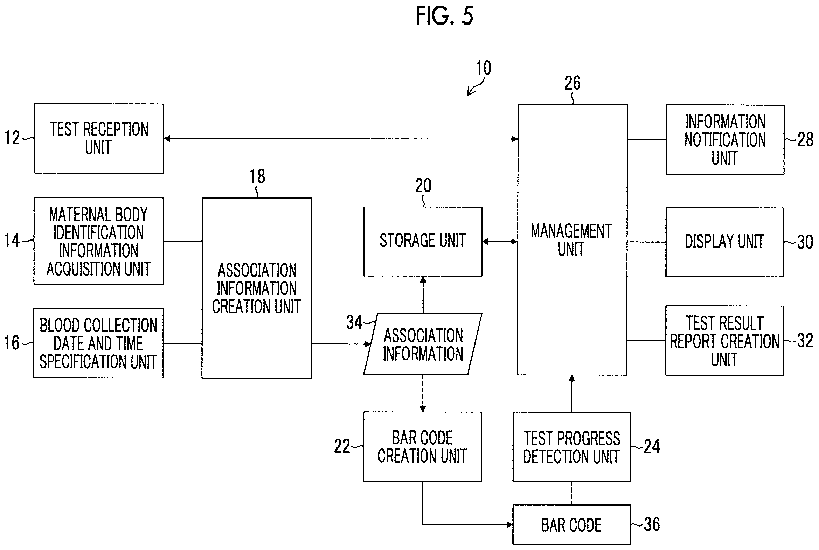

Fig. 5 is a block diagram in which an outline of a genetic chromosome test management system according to the present embodiment is shown. A genetic chromosome test management system 10 according to the present embodiment includes a test reception unit 12, a maternal body identification information acquisition unit 14, a blood collection date and time specification unit 16, an association information creation unit 18, a storage unit 20, a barcode creation unit 22, a test progress detection unit 24, a management unit 26, an information notification unit 28, a display unit 30, and a test result report creation unit 32. The maternal body identification information acquisition unit 14 is a form of an "identification information acquisition unit".

The genetic chromosome test management system 10 may be constructed as a client/server type computer system, or may be configured as a client system without using a server.

The test reception unit 12 is a processing unit which performs processing of receiving a request for a test. The test reception unit 12 can be set as, for example, a hospital client PC as a client terminal device installed in a hospital. The expression of a "PC" represents a personal computer, and various types of computers such as a desktop type computer, a notebook type computer, and a tablet type computer. The term "client PC" is used as a collective name of a terminal device. The test reception unit 12 receives a request for a test and transmits information of a test request with respect to the management unit 26.

The maternal body identification information acquisition unit 14 is means for acquiring identification information of a maternal body. The identification information of a maternal body is a number, a symbol string, a character string which are individually determined in order to specify a maternal body, or a unique identification code represented by appropriately combining them. As the identification information of a maternal body, it is possible to use, for example, a patient identification (ID).

The maternal body identification information acquisition unit 14 may be a hospital client PC or a server. Alternately, the maternal body identification information acquisition unit may be a test room client PC as a client terminal device installed in a test room in which a test work is executed. In the present specification, the hospital client PC is called a "hospital terminal" and the test room client PC is called a "test terminal".

When acquiring identification information of a maternal body, the maternal body identification information acquisition unit may be configured to acquire information through manual input such that, for example, an operator operates a keyboard or other input devices, or may be configured to receive information of an electronic medical chart from an electronic medical chart server. In addition, it is also possible to employ an aspect of independently issuing a unique ID in the genetic chromosome test management system 10. In a case where a unique ID which is different from a patient ID managed in a hospital is issued, the unique ID is associated with the patient ID (so-called linkage of information) within this system or in another system.

The blood collection date and time specification unit 16 is a processing unit which specifies the date and time at which blood is collected from a maternal body. The blood collection date and time specification unit 16 may be a hospital terminal or a test terminal. It is preferable that, if a prescribed operation such as pressing a test start button on a PC screen when, for example, executing blood collection is performed, the blood collection date and time specification unit is configured such that information of the blood collection date and time is automatically acquired in response to the operation.

The blood collection date and time indicates the date and time at which blood is collected from a maternal body. However, it is unnecessary to request strict specification of the actual collection time. The date and time specified as a blood collection date and time can be determined while including an error within an allowable time range before and after the actual collection time. For example, the time difference between pressing of the test start button and blood collection which is actually performed, the time difference between execution of blood collection and pressing of a "blood collection button" on a PC screen, or the like is treated as an error within an allowable time range.

As another configuration example of the blood collection date and time specification unit 16, the blood collection date and time specification unit 16 can be configured to determine the date and time, at which a request for a test is received, as a blood collection date and time. The date and time at which a request for a test is received can be set to a date and time at which a "blood collection button" is pressed on a PC screen after blood collection in a hospital. The date and time at which a request for a test is received can be set to a date and time at which a blood specimen is received in a test room. In a case where the date and time at which a blood specimen is received in a test room is employed as the blood collection date and time, it is a prerequisite that the time required for transportation of the blood specimen from a hospital to the test room is within a certain time which has been predetermined. Such an aspect can be employed since it is also possible to secure a certain time depending on an operation rule. In addition, it is also possible to secure the quality of the whole test if the test room is managed by an elapsed time from the date and time at which the blood specimen is received.

The association information creation unit 18 is a processing unit which associates identification information of a maternal body acquired in the maternal body identification information acquisition unit 14 with information of the blood collection date and time specified by the blood collection date and time specification unit 16 to create association information 34 showing a correspondence relation therebetween. The association information 34 created in the association information creation unit 18 is stored in the storage unit 20. The step of storing the identification information of a maternal body and the blood collection date and time in the storage unit 20 in association with each other corresponds to a form of a "storage step", and the function of storing the identification information of a maternal body and the blood collection date and time in the storage unit in association with each other corresponds to a form of a "storage function".

Each of the association information creation unit 18 and the storage unit 20 may be a hospital terminal, a test terminal, or a server.

It is possible to make the association information 34 stored in the storage unit 20 include information of a critical elapsed time from the blood collection date and time. The critical elapsed time defines an allowable upper limit which becomes a threshold value of a determination criterion for outputting an alert in a case where the elapsed time from the blood collection date and time exceeds the allowable upper limit. The critical elapsed time from the blood collection date and time to the completion of a specific step can be set to a predetermined value, for example, within 24 hours. In order to secure the number of nucleated red blood cells influencing the success or failure of a test, it is preferable to set the critical elapsed time after blood collection to be within 24 hours. In addition, a configuration in which the setting of the critical elapsed time is made to be variable can also be used. For example, the critical elapsed time is set for each layer of an attribution group classified according to the maternal body conditions such as the age of a maternal body, the number of weeks elapsed after pregnancy (referred to as the "number of weeks of pregnancy"), or a combination thereof.

The barcode creation unit 22 is means for creating a label of a barcode 36 used for identifying a specimen. In this example, a one-dimensional code is used. However, the term "barcode" is not limited to the one-dimensional code and also includes a two-dimensional code. At least one of identification information of a maternal body or information of a blood collection date and time is included in the barcode 36. The label of the barcode 36 is pasted to a part, such as a blood collection tube or slide glass, which is used for a test. The barcode 36 is preferably created simultaneously with collection of blood from a maternal body. The term "simultaneously" referred to herein includes a time difference within an allowable time range. In addition, the label of the barcode 36 can be newly and additionally created in a test room when a specimen is received in the test room.

It is possible to attract attention of a worker (test executor) of a test room by printing a date and time display indicating the blood collection date and time on the label of the barcode 36 in a readable notation form such as "year, month, day, hour, and minute". The barcode label in this case corresponds to a form of a "printed matter". A label in which a date and time display of the blood collection date and time is printed may be created separately from the label of the barcode 36.

The test progress detection unit 24 is means for detecting a progress condition of a test until at least a specific step is completed. The progress of the test can be ascertained and managed using the barcode 36. It is possible to use, for example, a barcode reader which is connected to a test terminal as the test progress detection unit 24. It is possible to incorporate barcode information and information of a read date and time by reading the barcode 36 at a start time or an end time of a process, or at both timings of the start time and the end time. The information obtained by reading the barcode 36 at a pause of steps can be utilized as information indicating a progress condition of a test.

The management unit 26 is a processing unit which manages a progress of a test until at least a specific step is completed, based on the progress condition detected by the test progress detection unit 24. The management includes accumulation of at least progress information in a database. The management unit 26 may be a hospital terminal, a test terminal, or a server. The management unit 26 can include a function of storing and updating information of an elapsed time until at least a specific step is completed while monitoring the elapsed time from the blood collection date and time.

The management unit 26 of this example has a function of managing the progress of a test of a blood specimen after blood collection, and manages the processing condition of the test, in which a test request is received, with respect to a plurality of specimens. The management unit 26 has a function of determining whether or not the test is completed based on the progress condition of the test in a case where the elapsed time has exceeded the critical elapsed time while comparing the critical elapsed time with the elapsed time from the blood collection date and time, and of notifying an operator of an alert and other pieces of excess information in a case where the test is not completed.

In a case of a configuration in which the setting of the critical elapsed time is made different according to the conditions of a maternal body, the management unit 26 can be configured to manage patient information including at least the age of the maternal body or the number of weeks of pregnancy, acquire a critical elapsed time based on the patient information, and compare the critical elapsed time with the elapsed time.

The information notification unit 28 is means for notifying an operator of excess information and other various pieces of information. The notification of excess information may be displayed on a screen of the management unit 26 or be displayed on a screen of a test terminal. In addition, it is possible to employ a configuration of notifying an operator of information using a sound instead of or in combination with the notification through display.

In a case where excess information is notified, the management unit 26 can perform processing of changing the order of tests of incomplete specimens in which tests are not completed. The information notification unit 28 corresponds to a form of a "notification unit".

The display unit 30 is display means for displaying various pieces of information managed by the management unit 26. It is possible to display the blood collection date and time or the elapsed time for each specimen, or both pieces of information thereof on the display unit 30. For example, even in a case where an alert is not output, the display of the blood collection date and time and/or the elapsed time of a specimen on a screen of a test terminal can become an opportunity of attracting attention of a worker in a test room.

In addition, it is possible to make the system be able to determine the order of tests after displaying a list with respect to a plurality of incomplete specimens on a screen of the display unit 30 and performing sorting (rearranging) based on the blood collection time or the elapsed time. When delivering blood specimens from a hospital to a test room, it is considered that a plurality of blood specimens are delivered together. Therefore, when starting a test after receiving the blood specimens in the test room, it is possible to determine the order of tests by performing sorting.

The test result report creation unit 32 is a processing unit which creates a report of test results. The test result report creation unit 32 creates a test result report in which test results are summarized in a report form. It is possible to make the test result report include information of the blood collection date and time and the test completion date and time. It is possible to make the test result report include information of an elapsed time from the blood collection date and time to the completion of a test, that is, information of total testing time after blood collection, instead of or in combination with the information of the blood collection date and time or the test completion date and time.

Furthermore, the management unit 26 can record the total testing time after blood collection together with the completion time of each step, in a system log. Using these pieces of information accumulated, it is possible to verify the quality of a test later.

For example, the recording can be used to ascertain whether or not nucleated red blood cells are efficiently collected or to ascertain the relation between the number of candidates of nucleated red blood cells which can be extracted in image analysis and the elapsed time after blood collection. Moreover, the recording can be used for determination as to whether how much time it will take to suitably complete steps.

In addition, the management unit 26 plays a role as means for managing whether a test request is acceptable or managing a reception schedule based on progress information of a test. That is, in a case where a test request is received from the test reception unit 12, the management unit 26 has a function of transmitting at least one of information pieces of whether the test request is acceptable, the progress condition of the test, or completion expectation of the test, to the test reception unit 12 based on the progress information of the test.

It is possible to make the progress information managed by the management unit 26 include booked information relating to a booked test which has not been started.

In the management unit 26, information of the number of specimens in which processing up until a specific step immediately before a step which is determined as not being affected by removal of nuclei is not completed is included in the progress information required for determination whether a test request is acceptable or for schedule management.

The management unit 26 stores a receivable threshold value up until a specific step immediately before a step which is determined as not being affected by removal of nuclei, as a setting value, and can be configured to determine that it is possible to receive a test request in a case where the number of incomplete specimens is smaller than a threshold value for the number of receivable specimens by comparing the threshold value for the number of receivable specimens with the number of incomplete specimens which is the number of specimens in which processing up until a specific step is not completed.

In addition, the system can be configured such that progress information of a step which is not affected by removal of nuclei is also considered together as another determination method. As a result of an examination of the present inventors, it has been found that it takes more time for processing or work of a step group which is not affected by removal of nuclei than that of a step group which is affected by removal of nuclei, in a series of test processes. For this reason, there is a concern that congestion of processing may occur in the step which is not affected by removal of nuclei if a test is received after determining whether the test request is acceptable using only the threshold value for the number of receivable specimens before the step which is not affected by removal of nuclei. It is possible to employ the following configuration as a countermeasure against such a problem.

That is, specimens remaining to be immobilized information relating to the number of specimens remaining to be immobilized in which processing up until an immobilization step which is the specific step immediately before a step which is determined as not being affected by removal of nuclei is not completed, and remaining incompleted testing specimens information relating to the number of remaining incompleted testing specimens in which processing of all steps of a genetic chromosome test is not completed are included as the progress information managed by the management unit 26. Moreover, a "threshold value for the number of immobilizable specimens" which is a threshold value for the number of receivable specimens having reached the immobilization step and a "threshold value for the number of specimens in which testing can be completed" which is a threshold value for the number of receivable specimens until processing of all steps of a genetic chromosome test is completed are set. The management unit 26 includes a function of determining whether test requests are acceptable by combining comparison results obtained by comparing the specimens remaining to be immobilized information with the threshold value for the number of immobilizable specimens and comparing the remaining incompleted testing specimens information with the threshold value for the number of specimens in which testing can be completed. In this case, the threshold value for the number of immobilizable specimens is set to a value greater than the threshold value for the number of specimens in which testing can be completed.

It is preferable to make each of the threshold values be changable through setting. However, during the setting in this case, a checking mechanism is placed so as to make the threshold value for the number of immobilizable specimens become a value greater than the threshold value for the number of specimens in which testing can be completed. For example, when a user inputs a value of the threshold value for the number of specimens in which testing can be completed, if the threshold value for the number of immobilizable specimens is a value smaller than the threshold value for the number of specimens in which testing can be completed by comparing the input value with the set threshold value for the number of immobilizable specimens, the value is not stored and resetting is promoted by displaying an error.

In addition, it is also possible to employ an aspect of determining whether a test request is acceptable from the viewpoint of time required for the test, as another determination method of whether a test request is acceptable. For example, the management unit 26 can be configured to store a standard testing time taken until a specific step and a receivable testing time threshold value as setting values, and determine that it is possible to receive a test request in a case where a remaining testing time, which is calculated by multiplying the standard testing time by the number of incomplete specimens in which processing up until the specific step is not completed, is smaller than the receivable testing time threshold value by comparing the remaining testing time with the receivable testing time threshold value.

In a case where there are a plurality of steps up until the specific step, the management unit can be configured to store a setting value of a standard testing time for each of these plurality of steps, and determine whether a test request is acceptable after calculating a remaining testing time from the number of incomplete specimens in each step using each standard testing time and comparing the calculated remaining testing time with the receivable testing time threshold value.

In addition, the management unit can be configured to include specimens remaining to be immobilized information relating to testing time for specimens remaining to be immobilized in which processing up until an immobilization step which is the specific step immediately before a step which is determined as not being affected by removal of nuclei is not completed, and remaining incompleted testing specimens information relating to testing time for remaining incompleted testing specimens in which processing of all steps of a genetic chromosome test is not completed, as progress information pieces managed by the management unit 26. Moreover, the management unit can be configured such that an "immobilizable testing time threshold value" which is a receivable testing time threshold value required to reach the immobilization step and a "completely testable testing time threshold value" which is a receivable testing time threshold value until processing of all steps of a genetic chromosome test is completed are set. In this case, the management unit 26 includes a function of determining whether test requests are acceptable by combining comparison results obtained by comparing the specimens remaining to be immobilized information with the immobilizable testing time threshold value and comparing the remaining incompleted testing specimens information with the completely testable testing time threshold value. In addition, the immobilizable testing time threshold value is set to a value greater than the completely testable testing time threshold value.

It is preferable to make each of the threshold values of the immobilizable testing time threshold value and the completely testable testing time threshold value be changable through setting similarly to the above-described threshold value for the number of immobilizable specimens and the threshold value for the number of specimens in which testing can be completed. In addition, the management unit preferably includes a function of automatically checking numerical values to be set such that the immobilizable testing time threshold value is a value greater than the completely testable testing time threshold value when performing setting or changing of threshold values.

The specimens remaining to be immobilized information relating to testing time for specimens remaining to be immobilized can be calculated from the number of specimens remaining to be immobilized and a standard testing time taken until the immobilization step. In addition, the remaining incompleted testing specimens information relating to testing time for remaining incompleted testing specimens in which processing of all steps of a genetic chromosome test is not completed can be calculated from the number of remaining incompleted testing specimens and a standard testing time taken until the completion of all steps.

In a case where it is determined that it is impossible to receive a test in determination whether a test request is acceptable (that is, in a case where it is determined that the reception is impossible), the management unit 26 has a function of calculating the date and time which is suitable and is recommended as a scheduled date and time of blood collection to perform blood collection from a maternal body and transmitting the calculated scheduled date and time of blood collection to the test reception unit 12. In the test reception unit 12, it is possible to adjust the time of blood collection based on the information of the scheduled date and time of blood collection notified from the management unit 26.

In addition, the management unit 26 can be configured to include a function of providing information of a completion expectation date and time of a test which has already been received, or a function of providing information of a completion expectation date and time of a new test in a case where a test is newly received, instead of or in combination with the function of determining whether a test request is acceptable.

In the test reception unit 12, a user himself or herself can spontaneously adjust a request for a test while checking such information of a completion expectation date and time.

The management method using the genetic chromosome test management system described in the present embodiment can be ascertained as a genetic chromosome test management method.

### <Specific Configuration Example of Genetic Chromosome Test Management System>

Next, a more specific embodiment will be described.

### «First Example»

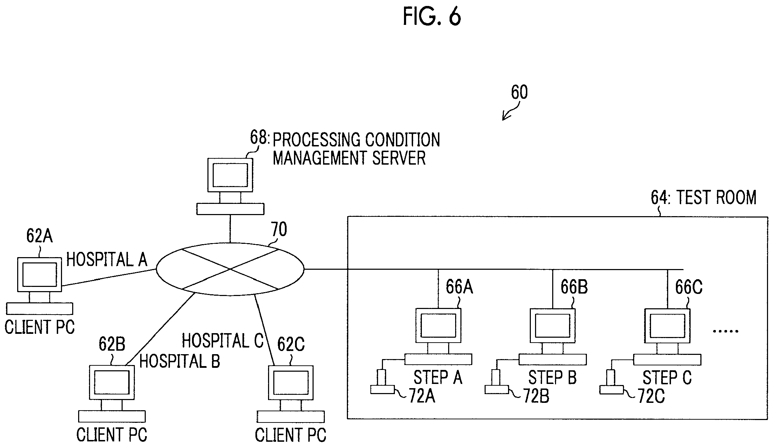

Fig. 6 is a configuration diagram of a genetic chromosome test management system 60 according to a first example. This genetic chromosome test management system 60 includes hospital terminals 62A to 62C installed in hospitals; test terminals 66A to 66C installed in a test room 64 in which a work of a specimen test is performed; and a processing condition management server 68 which manages the processing condition of a test process. The processing condition management server 68 is connected to the hospital terminals 62A to 62C and the test terminals 66A to 66C through an electric communication line 70.

The electric communication line 70 may be a local area network (LAN) or a wide area network (WAN), or a combination thereof. The electric communication line 70 is not limited to a wire communication line, and a part or the entirety of the electric communication line can be set to a wireless communication line. In addition, in the present specification, the notation "connection" between devices capable of transferring and receiving a signal to/from each other is not limited to wired connection, and also includes wireless connection.

In Fig. 6, three personal computers (PCs) of the hospital terminal 62A installed in a hospital A, a hospital terminal 62B installed in a hospital B, and a hospital terminal 62C installed in a hospital C are shown. However, the number of hospital terminals is not particularly limited when executing the invention, and can be set to an arbitrary number greater than or equal to 1. In addition, the system can be configured to include a plurality of hospital terminals in the same hospital.

In addition, in Fig. 6, a case in which there is one test room 64, but the number of test rooms 64 is not particularly limited, and can be set to an arbitrary number greater than or equal to 1. In this case, the processing condition management server 68 manages the progress condition for each of the plurality of test rooms 64.

The test room 64 receives work consignments of specimen tests from a plurality of hospitals A to C. Regarding the test terminals 66A to 66C provided in the test room 64, three PCs of the test terminal 66A used when performing processing of a step A in a test process, the test terminal 66B used when performing processing of a step B, and a test terminal 66C used when executing processing of a step C are representatively shown in Fig. 6. When performing the invention, the number of test terminals is not particularly limited, and can be set to an arbitrary number greater than or equal to 1. It is also possible to perform processing or progress detection of a plurality of steps in one test terminal.

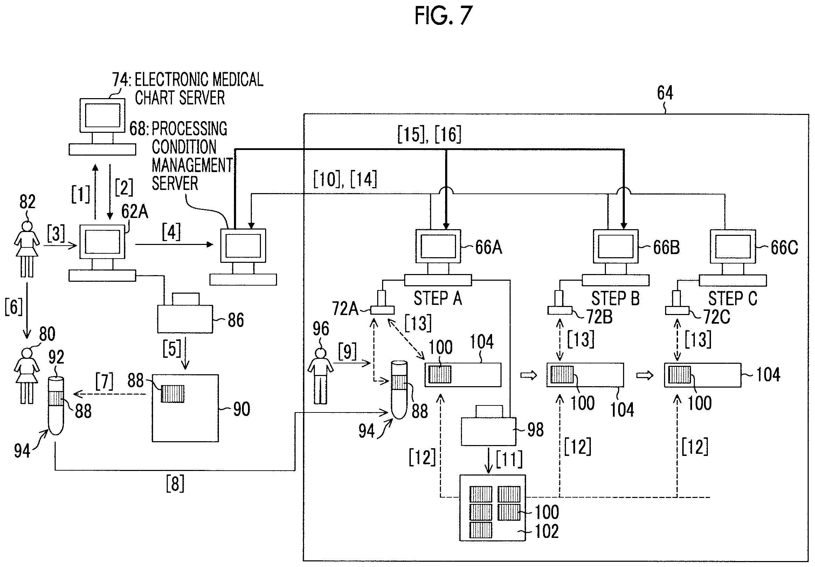

Barcode readers 72A to 72C are respectively connected to the test terminals 66A to 66C. Although is not shown in Fig. 6, a barcode including identification information for identifying a specimen is attached to a container storing blood or a slide glass (refer to Fig. 7), and reading of the barcode is performed by the barcode readers 72A to 72C when a work or processing of each step is started, or when a work or processing of a step is ended, or at both timings of the starting and the ending of a work or processing of a step.

The test terminals 66A to 66C can specify the date and time at which incorporation of barcode information is performed by the barcode readers 72A to 72C. The information read from the barcode readers 72A to 72C is notified to the processing condition management server 68. As described above, the method for dividing steps or the number of steps to be managed can be arbitrarily designed depending on the system.

The processing condition management server 68 corresponds to a form of a "storage unit", a "management unit", and a "test management server". The hospital terminals 62A to 62C correspond to a form of an "identification information acquisition unit", a "blood collection date and time specification unit", a "barcode creation unit", and a "first client terminal". The test terminals 66A to 66C including the barcode readers 72A to 72C correspond to a form of the "test progress detection unit" and the "second client terminal".

### <Flow of Execution of Test>

Fig. 7 is an explanatory view in which a state of executing a genetic chromosome test in the first example is schematically shown. Here, the hospital terminal 62A of the hospital A will be described as an example. The same principle also applies to the other hospital terminals 62B and 62C shown in Fig. 6.

In Fig. 7, a patient 80 who is to receive a genetic chromosome test is a pregnant woman. The patient 80 applies for a test through necessary procedures such as inquiry or counselling before the test. A medical practitioner 82 accesses an electronic medical chart server 74 by operating the hospital terminal 62A after receiving a test application from the patient 80. The medical practitioner 82 may be a co-medical such as a nurse or a test engineer, or may be a physician. In addition, medical practitioners 82 may be a plurality of people.

As shown by a code [1] of Fig. 7, a patient information request which requires patient information relating to the patient 80 is output to the electronic medical chart server 74 from the hospital terminal 62A. The electronic medical chart server 74 provides the patient information of the target patient 80 to the hospital terminal 62A according to the patient information request obtained from the hospital terminal 62A (refer to code [2]).

For example, a patient's name, a patient's ID, the number of weeks of pregnancy, age, or the like is included as the patient information. The patient's ID is a unique identification code defined by a figure, a symbol, a character, or appropriate combination thereof (hereinafter, collectively called a "character string") for each patient. The patient's ID has a meaning of anonymizing a patient, and is defined as a character string in which it is impossible to directly specify an individual.

The patient's ID corresponds to a form of an "identification information of a maternal body". The step of acquiring the patient's ID corresponds to a form of an "identification information acquisition step" and the function of acquiring the patient's ID corresponds to a form of an "identification information acquisition function".

The medical practitioner 82 checks patient information on a screen of the hospital terminal 62A and presses a test start button (not shown in the drawing) for starting a test (refer to a code [3]). The test start button can be configured as a graphical user interface (GUI) button shown on the screen of the hospital terminal 62A. "Pressing" or "pushing" of the GUI button means an input action of a command through a button operation such as a clicking operation or a touching operation, and does not always require a pushing action by physically adding a force. It is also possible to employ an aspect in which the test start button is constituted of a physical pressing switch or the like.

A test is started by the test start button being pressed, and then, management of steps is started. That is, if the test start button is pressed, information such as a patient's ID or a blood collection date and time is transmitted to the processing condition management server 68 from the hospital terminal 62A (refer to a code [4]). In this example, the date and time at which the test start button is pressed is treated as a "blood collection date and time". The hospital terminal 62A can ascertain the date and time using a clock built into a PC. The date and time at which the test start button is pressed is automatically specified as a "blood collection date and time".

The step of specifying a blood collection date and time corresponds to a form of a "blood collection date and time specification step", and the function of specifying a blood collection date and time corresponds to a form of a "blood collection date and time specification function".

Strictly speaking, time at which the test start button is pressed and time at which blood collection is actually performed are not always coincident with each other. However, it is considered that the time difference therebetween falls within a range of several minutes according to a general work procedure, which is an error range in which there is no particular operational problem. In addition, it is also considered that a work rule is determined as an operation rule of the system such that the time difference between pushing time of the test start button and actual blood collection execution time falls within a prescribed allowable range. In addition, it is also possible to employ a configuration of specifying time at which blood collection is actually executed by providing a blood collection date and time record button, a blood collection date and time input box, or the like separately from the test start button, as another aspect.

In a case of transmitting information relating to a patient to the processing condition management server 68 on the outside of the hospital from the hospital terminal 62A, it is preferable that information such as patient's name which can specify an individual is not provided while keeping the anonymity. That is, the information such as patient's name which can specify an individual is not transmitted, and a patient's ID using a character string or the like defined for each patient 80 is used as identification information of the patient 80. Association between the blood collection time and the identification information of the patient 80 in the hospital terminal 62A may be performed to store the relevant information, or the blood collection time and the identification information of the patient 80 in the processing condition management server 68 may be associated and stored.

A printer 86 is connected to the hospital terminal 62A, and printing of a barcode 88 is performed through the printer 86 in response to pushing of the test start button (refer to a code [5]). The barcode 88 is printed on label paper 90 for a barcode. The label of the barcode 88 printed on the label paper 90 can be pasted to a blood collection container 92 such as a blood collection tube as a seal label by being separated from release paper. In this manner, the barcode 88 is created simultaneously with a blood collection step in which blood is collected from the patient 80 who is a maternal body (within an allowable time range substantially considered as blood collection time). The barcode 88 corresponds to the barcode 36 described in Fig. 5.

It is preferable that the information included in the barcode 88 is anonymized. The barcode information embedded in the barcode 88 can be regarded as, for example, information of the patient's ID. In addition, as another example, it is possible to embed both information pieces of the patient's ID and the blood collection date and time in the barcode 88. It is also possible to employ a configuration in which the patient's ID is not included in the barcode information. It is also possible to employ an aspect in which, for example, only information of a blood collection date and time is embedded in the barcode 88 and the information of the patient's ID is omitted. In this case, association with a maternal body is performed based on the information of the blood collection date and time. In a case where there are a plurality of hospitals such as the hospitals A to C, an identifier which identifies a hospital is provided in the head of the information of the blood collection date and time.

In contrast, the medical practitioner 82 performs blood collection work of collecting blood from the patient 80 (refer to a code [6]). The label of the barcode 88 is pasted to the blood collection container 92 (refer to a code [7]). The label of the barcode 88 may be pasted to the blood collection container 92 after collecting peripheral blood of the patient 80 in the blood collection container 92, or blood of the patient 80 may be collected in the blood collection container 92 after pasting the label of the barcode 88 to the blood collection container 92.

A blood specimen 94 collected from the patient 80 is transported to the test room 64 (refer to a code [8]). In the test room 64, reception of the transported blood specimen 94 is performed. A worker 96 in the test room performs processing of the reception by reading the barcode 88 of the blood specimen 94 using the barcode reader 72A (refer to a code [9]). The test terminal 66A can specify the date and time at which barcode information is incorporated by the barcode reader 72A, and the date and time at which the barcode 88 of the blood specimen 94 is read is recorded in the test terminal 66A as a specimen reception date and time at which the barcode 88 of the blood specimen 94 is read. In addition, the barcode information and the information of a read date and time of a barcode which are read by the barcode reader 72A is notified from the test terminal 66A to the processing condition management server 68 (refer to a code [10]). That is, the patient's ID and the information of the specimen reception date and time are notified from the test terminal 66A to the processing condition management server 68 through the reading of the barcode 88 in the reception processing of the blood specimen 94.

In addition, a printer 98 is connected to the test terminal 66A, and if the barcode 88 during the reception of the blood specimen 94 is read, a printing command of a barcode 100 corresponding to the received blood specimen 94 is issued to the printer 98 from the test terminal 66A. The barcode 100 is printed by the printer 98 in response to this printing command (refer to a code [11]). The barcode 100 may have the same barcode information as barcode 88 of the blood specimen 94, or, if the association between both barcodes is secured, the barcode 100 may have barcode information different from the barcode 88.

The barcode 100 is printed on label paper 102 for a barcode plural times. The label of the barcode 100 which has been printed on the label paper 102 is pasted to a test part which treats blood specimen 94 (refer to a code [12]). A test tube, other containers, and the slide glass 104 are included in the test part. The work of pasting the label of the barcode 100 to the slide glass 104 is preferably performed immediately before preparing a smear sample or immediately after preparing a smear sample. By reading the barcode 100 attached to a container or the slide glass 104 using the barcode readers 72A to 72C during the start of and/or the completion of a work of each step (refer to a code [13]), the read barcode information or information of the read date and time are incorporated into the test terminals 66A to 66C.

Similarly to the test terminal 66A, the test terminals 66B and 66C can specify the date and time at which the incorporation of the barcode information is performed using respective the barcode readers 72B and 72C. The information of the date and time at which the incorporation of the barcode information is performed using the barcode readers 72B and 72C is notified to the processing condition management server 68 through the test terminals 66B and 66C.

By reading the barcode 100 using the barcode readers 72A to 72C during the start of and/or the completion of a work of each step (refer to a code [13]), patient's ID as specimen identification information, step identification information, and the date and time information at a work start time and/or a work completion time are notified from the test terminals 66A to 66C to the processing condition management server 68 (refer to a code [14]). These information pieces are information pieces showing progress conditions of a test process, and the notification of the specimen identification information, the step identification information, and the date and time information from the test terminals 66A to 66C to the processing condition management server 68 corresponds to notification of progress conditions of a test.

The step of detecting progress conditions of a test using read information pieces of the barcodes 88 and 100 corresponds to a form of a "test progress detection step" and a function of detecting progress conditions of a test using read information pieces of the barcodes 88 and 100 corresponds to a form of a "test progress detection function".

The step identification information is an identification code which is defined for each step in order to identify a plurality of steps to be managed. The step identification information may be a step number or a symbol such as A, B, and C, or a combination thereof.

In the example of Fig. 7, the test terminal 66A is used for detecting reception processing of the blood specimen 94 and progress conditions of a step range from a concentration step after the reception to the completion of a sample preparation step. The step range in which progress conditions are detected by the test terminal 66A is called a "step A".

The sample preparation step in the step A includes an immobilization step in which cells are immobilized. In a case where any step of a first drying step, a dyeing step, or a second drying step is determined as an immobilization step and steps after the step A including the immobilization step is determined as a step which is not affected by removal of nuclei, the step A is regarded as a specific step and a progress condition until the step A is completed is detected.

In addition, the test terminal 66B is used for detecting progress conditions of a step range from an acquisition step of a scan image using a slide scanner to completion of an image analysis step. The step range in which progress conditions are detected by the test terminal 66B is called a "step B". The test terminal 66C is used for detecting a progress condition of a genetic analysis step. The step range in which the progress condition is detected by the test terminal 66C is called a "step C". There is no possibility of removing nuclei in the steps B and C, and therefore, there have not been effects due to removal of nuclei.

The processing condition management server 68 manages progress information based on notification of progress conditions transmitted from the test terminals 66A to 66C. When the processing condition management server 68 receives information of a step start date and time showing a date and time at which a work of the step A with respect to the blood specimen 94 is started, the processing condition management server determines whether or not an alert is required by comparing this step start date and time with a blood collection date and time of the blood specimen 94. For example, an alert is generated (refer to a code [15]) in a case where the time difference (step start date and time - blood collection date and time) between the step start date and time and the blood collection date and time which is calculated exceeds a predetermined threshold value. The threshold value for determining whether or not an alert is required may be a fixed value which is previously set, or may be a value which is variably set according to the conditions of a maternal body. The notification of an alert may be performed through screen display of the processing condition management server 68, or may be performed through screen display of the test terminal 66A. The determination whether or not an alert is required may be performed on the test terminal 66A side.

In addition, as another example, the processing condition management server 68 can be configured to include a function of monitoring an elapsed time from the blood collection date and time for each blood specimen. For example, in the processing condition management server 68, a critical elapsed time is stored as a critical threshold value after blood collection, and the processing condition management server can be configured to regularly monitor whether or not the elapsed time from the blood collection date and time exceeds the critical elapsed time for each blood specimen. The processing condition management server 68 can be configured such that an alert is generated in a case where an elapsed time from the blood collection date and time exceeds the critical elapsed time. The critical elapsed time for determining whether or not an alert is required can be defined by relative time after the blood collection date and time. The critical elapsed time may be a predetermined fixed time, or a value which is variably set according to the conditions of a maternal body.

Furthermore, the processing condition management server 68 has a function of providing information of recommending the order of blood specimens to be used for starting tests with respect to a plurality of received blood specimens (refer to a code [16]). For example, the processing condition management server 68 has a function of generating an order list in which a priority order according to the number of weeks of pregnancy of a maternal body, the age, blood collection time, or the like is given to the plurality of blood specimens, and transmits the generated order list to the test terminal 66A.

The order list received from the processing condition management server 68 is displayed on the screen of the test terminal 66A. In the test room 64, it is possible to advance tests of blood specimens according to the priority order shown in the order list.

The processing condition management server 68 has a function of updating the order list by changing the test order according to progress conditions while monitoring an elapsed time after blood collection. The processing condition management server may be configured such that update of the order list displayed on the screen of the test terminal 66A is automatically performed, or the update may be performed according to a request from the test terminal 66A side.

The step of managing a progress of a test using the processing condition management server 68 corresponds to a form of a "management step" and the function of managing a progress of a test using the processing condition management server 68 corresponds to a form of a "management function".

According to the genetic chromosome test management system 60 of this example, it is possible to transfer and receive information of a patient's ID to/from the hospital A and the test room 64 online through the bar code 88 by transporting the blood specimen 94 to which the bar code 88 is attached. Accordingly, it is unnecessary to link a network between the hospital A and the test room 64, and therefore, it is possible to construct a safe system in view of management of information. In a case of particularly including information such as the age of a maternal body, it is preferable to transfer and receive to/from the hospital A and the test room 64 online.

### <Regarding Provision of Test Result>

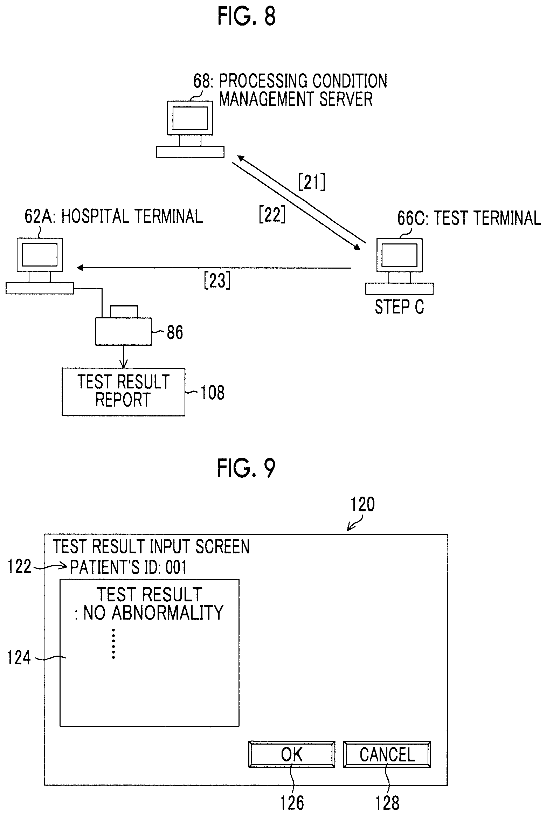

Fig. 8 is an explanatory view in which a state when providing a test result of the genetic chromosome test is schematically shown in the first example. Here, the hospital terminal 62A of the hospital A has been described as an example. The same principle also applies to the other hospital terminals 62B and 62C shown in Fig. 6.

In Fig. 8, an example in which a report of a test report is created by the test terminal 66C is shown. The test terminal 66C is a terminal used for a genetic analysis step, and is used for creating a report of a test result after analysis processing of the genetic analysis step is finished.

Fig. 9 is a view showing an example of a test result input screen displayed on the screen of the test terminal 66C. A test result input screen 120 includes a patient ID display unit 122 on which a patient's ID is displayed, a result entry column 124 for performing entry of test results, an OK button 126, and a cancel button 128.

A test executor inputs a test result to the result entry column 124 using a keyboard, a mouse, or other input devices which are not shown in the drawing. The result entry column 124 is not limited have a configuration of performing character input, and can be configured to have a entry support function that can select fixed entry contents from a pull-down menu or the like.

When the OK button 126 is pressed after results enter to the result entry column 124, the patient's ID is transmitted (refer to a code [21] of Fig. 8) to the processing condition management server 68 (refer to Fig. 8). When the cancel button 128 of Fig. 9 is pressed, the input processing of an input result is canceled.

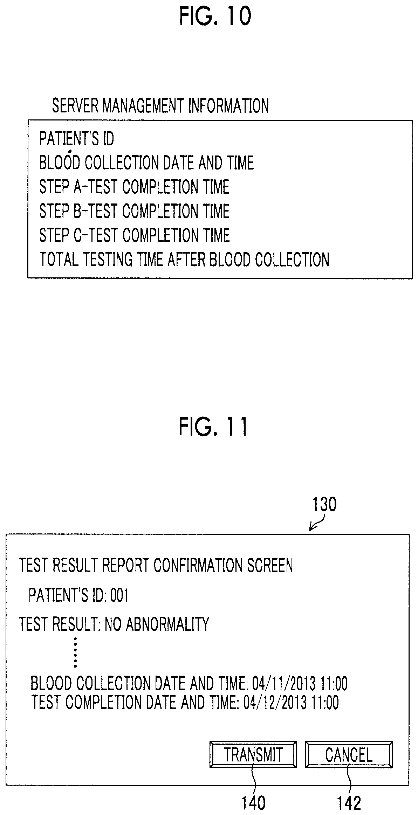

Fig. 10 is a view showing an example of information which is managed in a processing condition management server 68 (refer to Fig. 8). The processing condition management server 68 (refer to Fig. 8) manages each of the information pieces such as a blood collection date and time, step A-test completion time, step B test completion time, step C test completion time, and total testing time after blood collection, in association with a patient's ID. The total testing time may be information of a completion date and time at which all steps of a test are finished or information of required time until all steps are finished after blood collection, or a combination thereof.

When a patient's ID is transmitted to the processing condition management server 68 from the test terminal 66C of Fig. 8 (refer to a code [21]), the processing condition management server 68 answers information of a blood collection date and time and total testing time relating to the patient's ID with respect to the test terminal 66C (refer to a code [22]).

A confirmation screen of a test result report, in which information of a blood collection date and time and total testing time which are received from the processing condition management server 68 and information of test results are combined, is displayed on the screen of the test terminal 66C.

Fig. 11 is a view showing an example of a test result report confirmation screen displayed on the screen of the test terminal 66C (refer to Fig. 8). The patient's ID, the contents of a test result, a blood collection date and time, and a test completion date and time are displayed on a test result report confirmation screen 130. In addition, a transmit button 140 and a cancel button 142 are displayed on the test result report confirmation screen 130.

When the transmit button 140 is pressed after confirmation of report contents, information of the report is transmitted (refer to a code [23] of Fig. 8) to the hospital terminal 62A (refer to Fig. 8). When the cancel button 142 of Fig. 11 is pressed, the processing of providing a report is canceled.

It is also possible to display the contents of the test result report on the screen of the hospital terminal 62A shown in Fig. 8 and to create a printed matter 108 obtained by printing the contents of a test result report using a printer 86. By printing information of a test completion date and time in the printed matter 108 of the test result report, it is possible to check reliability of the test result. The printer 86 corresponds to a form of a "printing unit".

In Fig. 8, the example of transmitting the test result report from the test terminal 66C to the hospital terminal 62A online has been described. However, the method for providing a test result report is not limited to this example. For example, it is also possible to employ a configuration of providing a test result report to the hospital terminal 62A through the processing condition management server 68. In addition, it is also possible to employ a configuration of providing the test result report online after being printed by a print connected to the test terminal 66C.

### <Regarding Reception of Test>

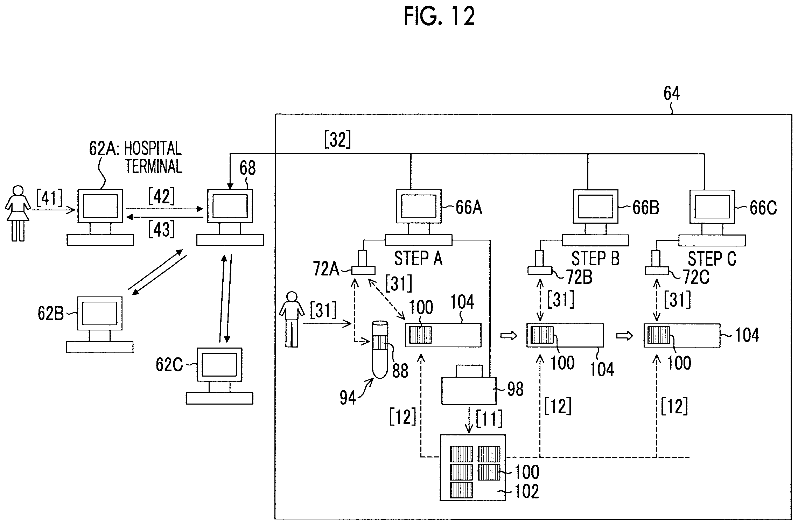

Fig. 12 is an explanatory view in which a state when a new test is requested in the configuration of the substituent in the first example is schematically shown. In Fig. 12, the same codes are given to the same elements as those in the configurations described in Figs. 6 and 7, and the description thereof will not be repeated. As described in Fig. 7, the processing condition management server 68 manages the progress of a test or processing conditions in the test room 64 based on notification of progress conditions of the test transmitted from the test terminals 66A to 66C.

In Fig. 12, if the barcode 88 or 100 is read (refer to a code [31]) during the start of each step after a new blood specimen 94 is received in the test room 64, information showing a progress condition of a test is notified to the processing condition management server 68 through the test terminals 66A to 66C (refer to a code [32]). The processing condition management server 68 ascertains and manages the progress of the test for each step and the overall processing condition based on the information notified from the test terminals 66A to 66C.

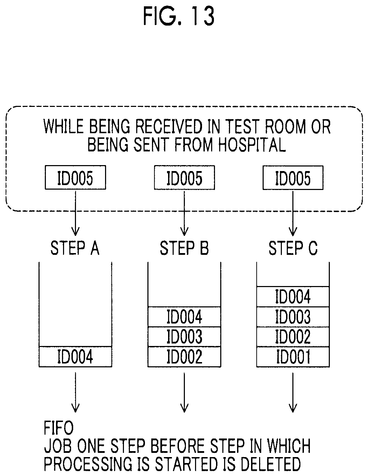

Fig. 13 is a conceptual view in which an example of a method for managing a progress of a test using the processing condition management server 68 is shown. Here, an example in which the progresses of three steps of the step A, the step B, and the step C are managed is shown.

Jobs of patient's IDs of incomplete specimens in which processing of steps A to C has not been finished are piled up on the division of each of the steps. The term "job" represents processing to be executed in the steps. That is, it is shown that the patient's IDs piled up on the division of each of the steps A to C in Fig. 13 are jobs of waiting for processing. For example, a job represented by "ID004" of the step A represents processing of the step A with respect to a patient's ID "ID004". In the case of Fig. 13, the number of incomplete specimens with respect to the step A is only one which is "ID004". The number of incomplete specimens with respect to the step B is three which are "ID002", "ID003, and "ID004". The number of incomplete specimens with respect to the step C is four which are "ID001 to "ID004".

In Fig. 13, patient's IDs of specimens which are newly received enter from the upper side of the division of each of the steps A to C, and processing is basically performed according to a rule of a first-in first-out (FIFO) method for each of the steps A to C. The jobs may be rearranged by providing a priority order depending on the conditions of a maternal body or the like. Processing is advanced in order from the step A, the step B, and the step C with respect to each specimen. In each of the steps A to C, a job one step before a step in which processing is started is deleted.

In the case of Fig. 13, the step A shows that the processing of "ID004" is started. The step B shows that the processing of "ID002" is started. The step C shows that the processing of "ID001" is started. Furthermore, in Fig. 13, a state in which reception of a test of "ID005" is newly performed under such a condition is shown. A job, which is newly added, is added to management of the processing condition management server 68 when being received in the test room 64 or when sending a specimen from a hospital. The management information of the progress condition of the processing in each of the steps A to C and the processing condition is updated anytime according to the reception of a test.

As described in Fig. 13, the processing condition management server 68 manages the processing condition of each of the steps A to C, and therefore, it is possible to provide information relating to the processing condition of a test to the hospital terminals 62A to 62C (refer to Fig. 12) or the like. It is possible to check the processing condition of the test room 64 in the hospital terminals 62A to 62C, and therefore, it is possible to adjust a request for a new test according to the processing condition.

The procedure when receiving a new test will be described while referring to Fig. 12. First, the medical practitioner 82 in the hospital A operates the hospital terminal 62A to inquire the processing condition of the test (refer to a code [41] of Fig. 12). Through the operation of this inquiry, a request is output from the hospital terminal 62A to the processing condition management server 68 (refer to a code [42] of Fig. 12). The processing condition management server 68 receives a request from the hospital terminal 62A to return a reply of information relating to the processing condition to the hospital terminal 62A (refer to a code [43] of Fig. 12). Information of a vacancy condition of the step A and a completion time up until the step C is included in the "information relating to the processing condition" which is sent from the processing condition management server 68 to the hospital terminal 62A. The "completion time up until the step C" corresponds to test completion-scheduled time showing completion expectation of a test, and may be a date and time of a test completion schedule or may be a length of time required up until the completion of a test.

It is possible to check the information relating to the processing condition on the screen of the hospital terminal 62A, and therefore, it is possible to adjust the request for a test. Moreover, it is possible to book a test from the hospital terminal 62A. Information of an order booked is transmitted from the hospital terminal 62A to the processing condition management server 68. The processing condition management server 68 performs registration of the booked order. When the booking of the test is performed, a job in a booked order is added to the management information of the processing condition described in Fig. 13 to update the management information of the processing condition.

### ] <Configuration Example of Processing Condition Management Server>

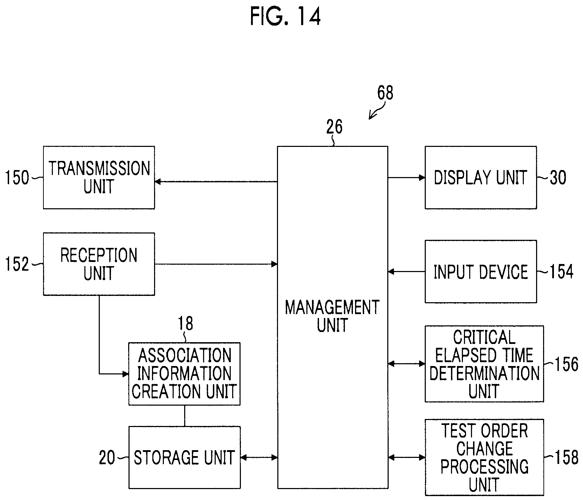

Fig. 14 is a block diagram showing a configuration example of the processing condition management server 68. In Fig. 14, the same codes are given to the same or similar elements as those in the configuration described in Fig. 5, and the description thereof will not be repeated.

The processing condition management server 68 includes a transmission unit 150, a reception unit 152, an input device 154, a critical elapsed time determination unit 156, and a test order change processing unit 158. The transmission unit 150 is means for transmitting various pieces of information to client terminals such as hospital terminals 62A to 62C or test terminals 66A to 66C described in Fig. 5. The reception unit 152 is means for receiving various kinds of information from the client terminals such as the hospital terminals 62A to 62C or the test terminals 66A to 66C.

That is, the transmission unit 150 and the reception unit 152 function as a communication interface unit for communicating with the client terminals. Identification information of a maternal body or information of a blood collection date and time are incorporated through the reception unit 152. In addition, detection information showing a progress condition of a test through the reception unit 152.

The input device 154 can employ various means such as a keyboard, a mouse, a touch panel, and a track ball, and these means may be appropriately combined. The input device 154 and the display unit 30 function as a user interface (UI).

The critical elapsed time determination unit 156 is a processing unit which performs processing of determining whether or not an elapsed time after blood collection exceeds the critical elapsed time, based on progress information managed by the management unit 26.

The test order change processing unit 158 is a processing unit which performs processing of changing the order of tests of blood specimens. The test order change processing unit 158 may automatically determine the order of tests from the priority order according to the conditions of a maternal body, or may change the order of tests according to an operation from the input device 154.

### <Configuration Example of Hospital Terminal>

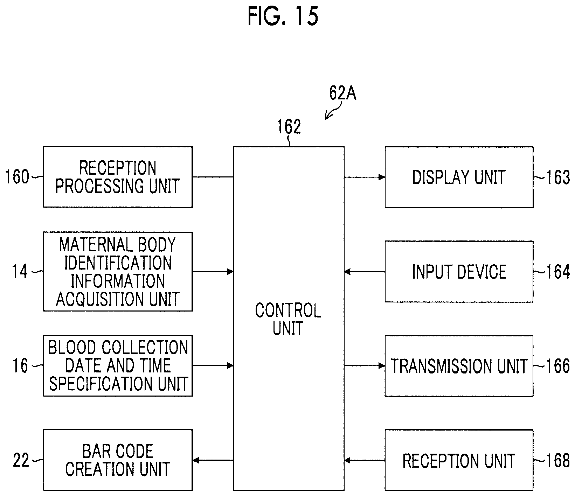

Fig. 15 is a block diagram showing a configuration example of the hospital terminal 62A. Although the hospital terminal 62A is shown here, the same principle also applies to the other hospital terminals 62B and 62C. In Fig. 15, the same codes are given to the same elements as those in the configuration described in Fig. 5, and the description thereof will not be repeated.

The hospital terminal 62A includes a reception processing unit 160, a control unit 162, a display unit 163, an input device 164, a transmission unit 166, and a reception unit 168. The reception processing unit 160 is means for carrying out procedural processing relating to reception of a test. The control unit 162 integrally controls various kinds of processing in the hospital terminal 62A. The display unit 163 and the input device 164 function as a user interface (UI). The input device 164 can employ various means such as a keyboard, a mouse, a touch panel, and a track ball, and these means may be appropriately combined. It is possible to employ a form in which the display unit 163 and the input device 164 are integrally configured, for example, a configuration in which a touch panel is disposed on a screen of the display unit 163.

The transmission unit 166 is means for transmitting various pieces of information to a server such as the processing condition management server 68 or the electronic medical chart server 74 described in Fig. 5. The reception unit 168 is means for receiving various kinds of information from the server such as the processing condition management server 68 or the electronic medical chart server 74. That is, the transmission unit 166 and the reception unit 168 function as a communication interface unit for performing communication with the server.

Identification information of a maternal body or information of a blood collection date and time are transmitted to the processing condition management server 68 through the transmission unit 166. In addition, it is possible to incorporate progress information relating to a progress condition of a test or information of a test result report through the reception unit 168.

### <Configuration Example of Test Terminal>

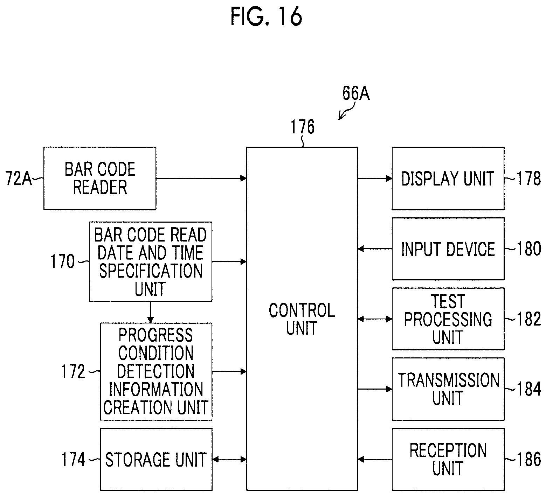

Fig. 16 is a block diagram showing a configuration example of the test terminal 66A. Although the test terminal 66A is shown here, the same principle also applies to the other test terminals 66B and 66C.

The test terminal 66A includes a barcode read date and time specification unit 170, a progress condition detection information creation unit 172, a storage unit 174, a control unit 176, a display unit 178, an input device 180, a test processing unit 182, a transmission unit 184, and a reception unit 186.

] The barcode read date and time specification unit 170 is a processing unit which specifies information of a date and time at which reading of barcode information is executed using the barcode reader 72A. The progress condition detection information creation unit 172 is a processing unit which creates detection information showing a progress condition of a test. For example, detection information including a patient's ID, identification information of steps, and information such as a work start time of a step or the like is generated based on the read barcode information. The storage unit 174 is means for storing various pieces of information. Identification information of steps, various programs, or the like are stored in the storage unit 174.

The control unit 176 integrally controls various kinds of processing in the test terminal 66A. The display unit 178 and the input device 180 functions as a user interface (UI). The input device 180 can employ various means such as a keyboard, a mouse, a touch panel, and a track ball, and these means may be appropriately combined.

The test processing unit 182 is a processing unit which performs various kinds of arithmetic processing or work support in steps of tests. The test processing unit 182 carries out processing, for example, mass calculation, component analysis, image analysis processing, or genetic analysis processing.

The transmission unit 184 is means for transmitting various kinds of information to a server such as the processing condition management server 68 described in Fig. 5. The reception unit 186 is means for receiving various kinds of information from the server such as the processing condition management server 68. The transmission unit 184 and the reception unit 186 function as a communication interface unit for performing communication with the server or other client terminals.

Detection information showing a progress condition of a test is transmitted to the processing condition management server 68 through the transmission unit 184. In addition, excess information or the like is acquired from the processing condition management server 68 through the reception unit 186.

### «Second Example»

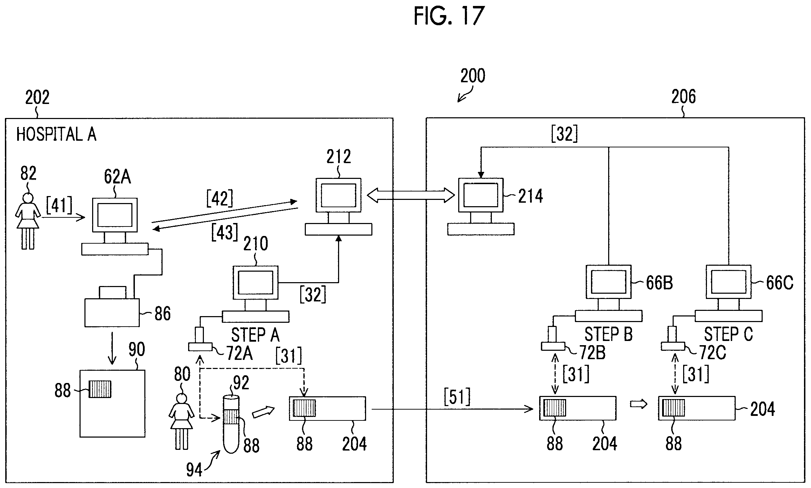

Fig. 17 is a configuration view of a genetic chromosome test management system 200 according to a second example. In Fig. 17, the same codes are given to the same or similar elements as those in the configurations of the first example described in Figs. 6 to 16, and the description thereof will not be repeated. Here, the difference between the second example and the first example which has been described in Fig. 7 will be described.

As shown in Fig. 17, the genetic chromosome test management system 200 according to the second example is a system in a case where steps up until preparation of a smearing sample is finished in a hospital 202, and a barcode-attached slide 204 which is the smearing sample is transported to a test room 206.

In this genetic chromosome test management system 200, a test terminal 210 and a first processing condition management server 212 are installed in the hospital 202 and a second processing condition management server 214 is installed in the test room 206. The test terminal 210 has the same function as that of the test terminal 66A described in Fig. 5. When the blood specimen 94 is received, the test terminal is used for detecting a progress condition of a test until processing of a step A is completed.

The first processing condition management server 212 and the second processing condition management server 214 communicate with each other, and information pieces of test conditions managed by both servers are synchronized. In the case of the configuration of this system, a side on which an ID for identifying the blood specimen 94 is issued becomes the hospital. Therefore, the information of the ID is synchronized by being transmitted from the first processing condition management server 212 to the second processing condition management server 214 of the test room 206 side.

The barcode-attached slide 204 in which the processing of the step A is completed in the hospital 202 is transported to the test room 206 (refer to a code [51]). In the test room 206, the barcode-attached slide 204 is received and processing of a step B and a step C is performed. Detection information showing a progress condition of each step is notified to the second processing condition management server 214 through the test terminals 66B and 66C. By employing such a configuration in the second processing condition management server 214, it is possible to shorten transportation time of blood test to the test room 64, and therefore, it is possible to promptly complete the processing up until the step A which is affected by removal of nuclei, after blood collection.

Each of the first processing condition management server 212 and the second processing condition management server 214, or a combination thereof corresponds to a form of a "management unit".

### «Third Example»

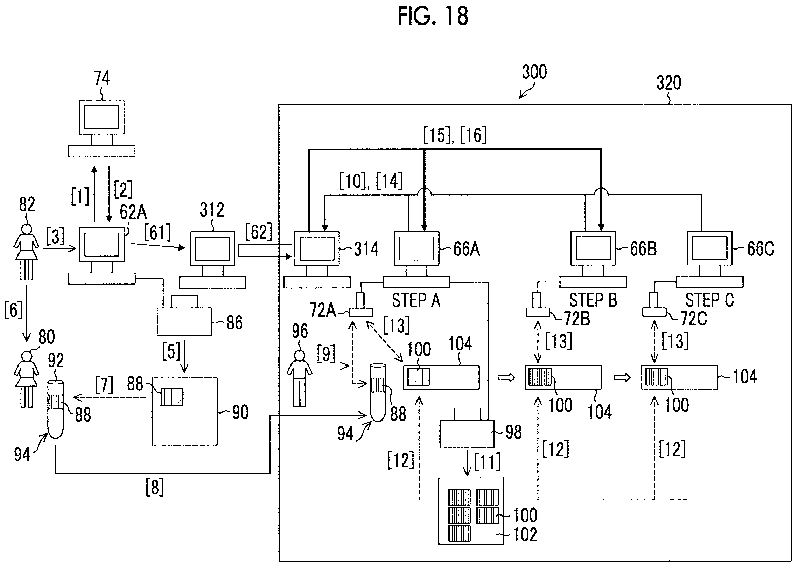

Fig. 18 is a configuration view of a genetic chromosome test management system 300 according to a third example. In Fig. 18, the same codes are given to the same or similar elements as those in the configurations of the first example described in Figs. 6 to 16, and the description thereof will not be repeated. Here, the difference between the third example and the first example which has been described in Fig. 12 will be described.

The genetic chromosome test management system 300 shown in Fig. 18 includes a blood collection date and time management server 312 which manages the blood collection date and time and a processing condition management server 314. The blood collection date and time management server 312 receives information of a blood collection date and time from the hospital terminal 62A (refer to a code [61]) and stores identification information of a maternal body and the blood collection date and time in association with each other. It is sufficient for the blood collection date and time management server 312 to manage only the identification information of a maternal body and the blood collection date and time.

The processing condition management server 314 has a function of managing a progress of a test based on detection information showing a progress condition of the test notified from the test terminals 66A to 66C similarly to the processing condition management server 68 described in Fig. 7. In addition, the processing condition management server 314 can acquire information of a blood collection date and time from the blood collection date and time management server 312 (refer to a code [62]) by communicating with the blood collection date and time management server 312 as necessary. The processing condition management server 314 transmits a patient's ID to the blood collection date and time management server 312 to inquire a blood collection date and time. The blood collection date and time management server 312 provides information of the blood collection date and time of the patient's ID which has been inquired, to the processing condition management server 314. The blood collection date and time management server 312 may be provided inside or outside a hospital. The processing condition management server 314 can be provided in a test room 320 as shown in Fig. 18.

In the genetic chromosome test management system 300 of this example, it is unnecessary to embed information of a blood collection date and time in barcode information of the barcode 88 printed by the printer 86 of the hospital terminal 62A, and it is possible to omit the information of a blood collection date and time while embedding information of a patient's ID as the barcode information.

In the test room 320 in which the blood specimen 94 to which the barcode 88 is attached is received, the information of a blood collection date and time is acquired from the blood collection date and time management server 312 using the patient's ID.

] According to such a configuration, it is possible to refer to the information of the blood collection date and time anytime on the test room 320 side, and therefore, it is possible to comparatively freely construct the system on the test room 320 side.

In addition, according to the genetic chromosome test management system 300 of this example, the information of the patient's ID is transferred and received between the hospital A and the test room 320 through the barcode 88 by transporting the blood specimen 94 to which the barcode 88 is attached. For this reason, it is unnecessary to link a network between the hospital A and the test room 320, and therefore, this system is safer than others also in view of management of information.

The blood collection date and time management server 312 corresponds to a form of a "storage unit". The processing condition management server 314 corresponds to a form of a "management unit".

### <Modification Example 1>

Execution of the invention is not limited to the above-described example, and various kinds of modification can be made. For example, the hospital terminal 62A and the processing condition management server 68 described in Fig. 5 or the like can be set as the same PC.

In addition, the hospital terminal 62A and the test terminal 210 described in Fig. 17 can be set to be the same PC. Furthermore, the first processing condition management server 212 and the second processing condition management server 214 described in Fig. 17 can be set to be the same PC. The test terminals 66A to 66C and other client PCs or the processing condition management server 68, the second processing condition management server 214, or the like can also be set to be the same PC.

Terminals may be divided into a plurality of PCs or may be provided in the same PC as long as the terminals are within at least the same facility such as in the same hospital or the same test room.

### <Modification Example 2>

In the genetic chromosome test, it takes comparatively more time to perform processing after an image processing step which is a step after a step of preparing a slide as a smearing sample is completed. The reason why it takes time for the image processing step is that, for example, the amount of data of an image read from the slide is extremely large. That is, in a test which is advanced in order of a step A, a step B, and a step C, the steps after the step B becomes a bottleneck. Accordingly, the system is in a preferred form having a configuration in which it is possible to perform a plurality of tests in parallel, with respect to test steps after the step B including a slide scan step or an image analysis step.

By setting facilities in a test room so as to simultaneously perform processing on works after the step B with respect to a plurality of blood specimens, it is possible to shorten a test completion time.

### <Modification Example 3>

In the above-described embodiment, it is also possible to employ a form of using radio frequency identification (RFID) instead of using a barcode or in combination with the use of a barcode. The label of a barcode can be replaced with a label of an RF tag, and the barcode reader can be replaced with an RFID reader.

### <Advantage of Embodiment>

(1) According to the embodiment of above-described invention, it is possible to manage a blood collection date and time and to manage a progress of a test based on the blood collection time. Therefore, it is possible to secure or ascertain the accuracy of the success or failure of a test.
(2) In addition, according to the embodiment of the present invention, even in a case where different systems are constructed for each test room, the registered blood collection dates and times are unified without depending on the test rooms. Therefore, it is possible to maintain the quality of tests constant.
   That is, even in either case where different systems are constructed for each test room or the same system is constructed for each test room, it is possible to secure the accuracy of the success or failure of the test of the above-described (1) regardless of the form of the system.
(3) By printing information of a blood collection date and time on a label of a barcode to be attached to a container of a blood specimen or slide glass in a notation format which is readable by a person, a test worker can advance a work while checking the blood collection date and time on the label. According to the aspect, it is possible to attract attention of the test worker so as to complete a test work within a prescribed time, and therefore, it is possible to secure the quality of the test.
(4) According to the embodiment of the present invention, it is possible to provide information relating to determination of whether a new test request is acceptable or to reception schedule by detecting a progress condition of a test and managing a processing condition of a test room. Accordingly, it is possible to secure the quality of the test.

### <Regarding Computer Program>

It is possible to provide a program for causing a computer to realize a genetic chromosome test management function of the genetic chromosome test management system described in the above-described embodiment, through an information storage medium by recording the program in a CD-ROM, a magnetic disk, or other computer readable media (non-transitory information storage media as tangible objects). In addition, it is also possible to provide a program signal as a download service using a communication network such as Internet instead of the aspect of providing the program by being stored in the information storage medium.

In addition, in a case where the genetic chromosome test management system is configured as a client/server type system, it is possible to provide a program for causing a computer to function as a client terminal or a server which constitutes the genetic chromosome test management system, through an information storage medium by recording the program in a CD-ROM, a magnetic disk, or other computer readable media (non-transitory information storage media as tangible objects).

By incorporating such a program into a computer, it is possible to cause a computer to function as the genetic chromosome test management system or to function as the client terminal of the genetic chromosome test management system or the test management server.

The present invention is not limited to the embodiment described above, and various kinds of modification can be made by possession of general knowledge in this technical field within technical ideas of the present invention.

### Explanation of References

- 10:: genetic chromosome test management system
- 14:: maternal body identification information acquisition unit
- 16:: blood collection date and time specification unit
- 20:: storage unit
- 24:: test progress condition detection unit
- 26:: management unit
- 28:: information notification unit
- 30:: display unit
- 36:: barcode
- 60:: genetic chromosome test management system
- 62A, 62B, 62C:: hospital terminal
- 64:: test room
- 66A, 66B, 66C:: test terminal
- 68:: processing condition management server
- 72A, 72B, 72C:: barcode reader
- 86:: printer
- 88:: barcode
- 92:: blood collection container
- 94:: blood specimen
- 98:: printer
- 100:: barcode
- 104:: slide glass
- 108:: printed matter
- 200:: genetic chromosome test management system
- 210:: test terminal
- 212:: first processing condition management server
- 214:: second processing condition management server
- 300:: genetic chromosome test management system
- 312:: blood collection date and time management server
- 314:: processing condition management server

## Claims

1. A genetic chromosome test management system used for a genetic chromosome test for testing an abnormality of a chromosome contained in fetus-derived nucleated red blood cells, the genetic chromosome test management system comprising:
an identification information acquisition unit which acquires identification information of a maternal body;
a blood collection date and time specification unit which specifies a date and time at which blood is collected from the maternal body;
a storage unit which stores the identification information of the maternal body acquired from the identification information acquisition unit and information of the blood collection date and time specified by the blood collection date and time specification unit in association with each other;
a test progress detection unit which detects a progress condition of a test until at least a specific step is completed in a case where a step one step before a step which is determined as not being affected by removal of nuclei among a plurality of steps successively executed in the genetic chromosome test is regarded as the specific step; and
a management unit which manages a progress of the test until at least the specific step is completed, based on the progress condition detected by the test progress detection unit.

2. The genetic chromosome test management system according to claim 1,
wherein the management unit manages an elapsed time from the blood collection date and time.

3. The genetic chromosome test management system according to claim 2,
wherein the management unit manages the progress of the test of a blood specimen collected from the maternal body.

4. The genetic chromosome test management system according to claim 3,
wherein the management unit manages at least the progress of the testing up until a step of immobilizing fetus-derived nucleated red blood cells among a plurality of steps, and the elapsed time from the blood collection date and time.

5. The genetic chromosome test management system according to claim 3,
wherein the management unit manages at least the progress of the testing up until a step of extracting deoxyribonucleic acid from the fetus-derived nucleated red blood cells, and the elapsed time from the blood collection date and time.

6. The genetic chromosome test management system according to any one of claims 3 to 5,
wherein a critical elapsed time determined as a critical threshold value which is allowable as the elapsed time after the blood collection is stored in the storage unit,
wherein the management unit determines whether or not the test is completed based on the progress condition of the test in a case where the elapsed time exceeds the critical elapsed time by comparing the elapsed time with the critical elapsed time, and
wherein the genetic chromosome test management system further comprises a notification unit which notifies excess information showing that the elapsed time has exceeded the critical elapsed time in a case where it is considered that the test has not been completed from the determination of the management unit.

7. The genetic chromosome test management system according to claim 6,
wherein, in a case where the excess information is notified, the management unit can change the order of tests of a blood specimen in which the tests are not completed.

8. The genetic chromosome test management system according to claim 6 or 7,
wherein the critical elapsed time is set for each layer which is classified according to at least one of the maternal body conditions of the age of the maternal body or the number of weeks elapsed after pregnancy, and
wherein the management unit manages patient information including at least one of the age of the maternal body or the number of weeks elapsed after pregnancy, acquires the critical elapsed time based on the patient information, and compares the critical elapsed time with the elapsed time.

9. The genetic chromosome test management system according to any one of claims 3 to 8,
wherein the progress of the test of the blood specimen is managed by a barcode, and
wherein the barcode is created when blood is collected from the maternal body.

10. The genetic chromosome test management system according to claim 9,
wherein at least one of information pieces of the blood collection date and time or identification information of the maternal body is included in the barcode.

11. The genetic chromosome test management system according to any one of claims 2 to 10, further comprising:
a display unit which displays at least one of the blood collection date and time or the elapsed time.

12. The genetic chromosome test management system according to any one of claims 1 to 11, further comprising:
a printing unit which creates a printed matter in which the information of the blood collection date and time is printed.

13. The genetic chromosome test management system according to any one of claims 1 to 12,
wherein the blood collection date and time specification unit specifies a date and time at which a request for a test is received as the blood collection date and time.

14. A test management server used for a genetic chromosome test for testing an abnormality of a chromosome contained in fetus-derived nucleated red blood cells, comprising:
a reception unit which receives identification information of a maternal body and information of a blood collection date and time at which blood is collected from the maternal body, from a first client terminal;
a storage unit which stores the identification information of the maternal body and the information of the blood collection date and time in association with each other; and
a management unit which receives detection information showing a progress condition of a test from a second client terminal including a test progress detection unit detecting the progress condition of the test until at least the specific step is completed in a case where a step one step before a step which is determined as not being affected by removal of nuclei among a plurality of steps successively executed in the genetic chromosome test is regarded as the specific step, and manages a progress of the test until at least the specific step is completed, based on the detection information.

15. A client terminal used for a genetic chromosome test for testing an abnormality of a chromosome contained in fetus-derived nucleated red blood cells, comprising:
an identification information acquisition unit which acquires identification information of a maternal body;
a blood collection date and time specification unit which specifies a date and time at which blood is collected from the maternal body; and
a transmission unit which transmits the identification information of the maternal body and the information of the blood collection date and time to a test management server, which manages the identification information of the maternal body acquired from the identification information acquisition unit and the information of the blood collection date and time specified by the blood collection date and time specification unit, and manages a progress of a test until at least a specific step is completed in a case where a step one step before a step which is determined as not being affected by removal of nuclei among a plurality of steps successively executed in the genetic chromosome test is regarded as the specific step.

16. A client terminal used for a genetic chromosome test for testing an abnormality of a chromosome contained in fetus-derived nucleated red blood cells, comprising:
a test progress detection unit which detects a progress condition of a test until at least a specific step is completed in a case where a step one step before a step which is determined as not being affected by removal of nuclei among a plurality of steps successively executed in the genetic chromosome test is regarded as the specific step; and
a transmission unit which transmits information of the progress condition detected by the test progress detection unit to a test management server managing a progress of the test until at least the specific step is completed, based on the progress condition detected by the test progress detection unit.

17. A genetic chromosome test management method for managing a genetic chromosome test for testing an abnormality of a chromosome contained in fetus-derived nucleated red blood cells, comprising:
an identification information acquisition step of acquiring identification information of a maternal body;
a blood collection date and time specification step of specifying a date and time at which blood is collected from the maternal body;
a storage step of storing the identification information of the maternal body acquired through the identification information acquisition step and information of the blood collection date and time specified through the blood collection date and time specification step in association with each other;
a test progress detection step of detecting a progress condition of a test until at least a specific step is completed in a case where a step one step before a step which is determined as not being affected by removal of nuclei among a plurality of steps successively executed in the genetic chromosome test is regarded as the specific step; and
a management step of managing a progress of the test until at least the specific step is completed, based on the progress condition detected through the test progress detection step.

18. A program for causing a computer to realize the genetic chromosome test management method according to claim 17.

19. A program for causing a computer to realize a genetic chromosome test management function used for a genetic chromosome test for testing an abnormality of a chromosome contained in fetus-derived nucleated red blood cells, the program causing the computer to realize:
an identification information acquisition function of acquiring identification information of a maternal body;
a blood collection date and time specification function of specifying a date and time at which blood is collected from the maternal body;
a storage function of storing the identification information of the maternal body acquired from the identification information acquisition function and information of the blood collection date and time specified using the blood collection date and time specification function in association with each other;
a test progress detection function of detecting a progress condition of a test until at least a specific step is completed in a case where a step one step before a step which is determined as not being affected by removal of nuclei among a plurality of steps successively executed in the genetic chromosome test is regarded as the specific step; and
a management function of managing a progress of the test until at least the specific step is completed, based on the progress condition detected using the test progress detection function.
